(19) Europäisches Patentamt
European Patent Office
Office européen des brevets

(11) Veröffentlichungsnummer : **0 392 241 B1**

(12) # EUROPÄISCHE PATENTSCHRIFT

(45) Veröffentlichungstag der Patentschrift : **02.06.93 Patentblatt 93/22**

(51) Int. Cl.$^5$ : **C07D 231/44,** A01N 43/56, C07D 401/04, A01N 43/40

(21) Anmeldenummer : **90105657.2**

(22) Anmeldetag : **24.03.90**

(54) **Substituierte 1-Arylpyrazole.**

Die Akte enthält technische Angaben, die nach dem Eingang der Anmeldung eingereicht wurden und die nicht in dieser Patentschrift enthalten sind.

(30) Priorität : **08.04.89 DE 3911556**

(43) Veröffentlichungstag der Anmeldung : **17.10.90 Patentblatt 90/42**

(45) Bekanntmachung des Hinweises auf die Patenterteilung : **02.06.93 Patentblatt 93/22**

(84) Benannte Vertragsstaaten : **BE CH DE FR GB IT LI NL**

(56) Entgegenhaltungen :
**EP-A- 234 119**
**EP-A- 235 628**
**EP-A- 0 201 852**
**EP-A- 0 301 339**
**EP-A- 0 320 750**

(73) Patentinhaber : **BAYER AG**
**W-5090 Leverkusen 1 Bayerwerk (DE)**

(72) Erfinder : **Jensen-Korte, Uta, Dr.**
**Geibelstrasse 9**
**W-4000 Düsseldorf 1 (DE)**
Erfinder : **Schallner, Otto, Dr.**
**Noldeweg 22**
**W-4019 Monheim (DE)**
Erfinder : **Becker, Benedikt, Dr., c/o Versuchsstation für Pflanzenschutz, Steinmannhof**
**I-39050 Pineta di Laives, Bolzano (IT)**
Erfinder : **Hartwig, Jürgen, Dr.**
**Franz-Esser-Strasse 44**
**W-5090 Leverkusen 3 (DE)**
Erfinder : **Stendel, Wilhelm, Dr.**
**In den Birken 55**
**W-5600 Wuppertal 1 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue substituierte 1-Arylpyrazole, mehrere Verfahren zu ihrer Herstellung und ihre Verwendung als Schädlingsbekämpfungsmittel.

Es ist bekannt, daß bestimmte substituierte 1-Arylpyrazole, wie beispielsweise die Verbindung 5-Amino-1-(2,6-dichlor-4-trifluormethylphenyl)-4-methylthiopyrazol oder die Verbindung 5-Amino-1-(2,6-dichlor-4-trifluormethylphenyl-3-methyl-4-dichlorfluormethylthiopyrazol oder die Verbindung 1-(2,6-Dichlor-4-trifluormethylphenyl)-3-methyl-4-dichlorfluormethylsulfonyl-5-propionamidopyrazol oder die Verbindung 5-Amino-1-(2,6-dichlor-4-trifluormethylphenyl)-3-methyl-4-dichlorfluormethylsulfinyl-pyrazol oder die Verbindung 5-Amino-1-(2,6-dichlor-4-trifluormethylphenyl)-4-trifluormethylsulfinyl-pyrazol gute Wirksamkeit gegen Schädlinge besitzen (vgl. z.B. EP-A 201 852).

Weiterhin wird die Verwendung von N-Phenylpyrazolen als Pestizide in der EP-A 0 234 119 beschrieben.

Die Wirkungshöhe bzw, Wirkungsdauer dieser vorbekannten Verbindungen ist jedoch, insbesondere bei bestimmten Insekten oder bei niedrigen Anwendungskonzentrationen nicht in allen Anwendungsgebieten völlig zufriedenstellend.

Es wurden neue substituierte 1-Arylpyrazole der allgemeinen Formel (I),

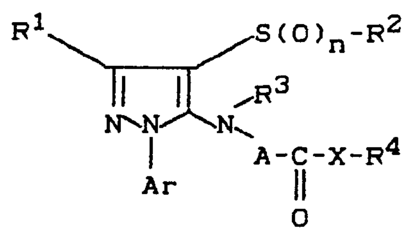

(I)

in welcher

$R^1$ für Wasserstoff oder für geradkettiges oder verzweigtes Alkyl mit 1 bis 6 Kohlenstoffatomen steht,

$R^2$ für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^3$ für Wasserstoff oder für geradkettiges oder verzweigtes Alkanoyl mit 1 bis 5 Kohlenstoffatomen steht,

$R^4$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
und außerdem für den Fall, daß X für Sauerstoff oder Schwefel steht, auch für ein Äquivalent eines Alkali-, Erdalkali-, Kupfer-, Zink-, Mangan-, Zinn-, Eisen-Cobalt- oder Nickelkations oder für ein gegebenenfalls einfach oder mehrfach, gleich oder verschieden substituiertes Ammonium-, Phosphonium-oder Sulfoniumkation steht, wobei als Substituenten infrage kommen: geradkettiges oder verzweigtes Alkyl mit 1 bis 18 Kohlenstoffatomen, Phenyl oder Benzyl,

A für einen zweifach verknüpften, geradkettigen oder verzweigten Alkylenrest mit 1 bis 12 Kohlenstoffatomen steht,

X für Sauerstoff, Schwefel oder für einen Rest

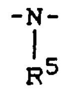

steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^6$ und,

n für eine Zahl 0, 1 oder 2 steht, wobei

$R^5$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^6$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht,

gefunden.

Weiterhin wurde gefunden, daß man die neuen substituierten 1-Arylpyrazole der allgemeinen Formel (I),

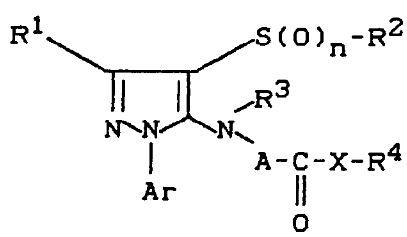

(I)

in welcher

$R^1$, $R^2$, $R^3$, $R^4$, A, X, Ar und n die oben angegebenen Bedeutungen haben,

nach einem der im Folgenden beschriebenen Verfahren erhält:

(a) Man erhält substituierte 1-Arylpyrazole der Formel (I),

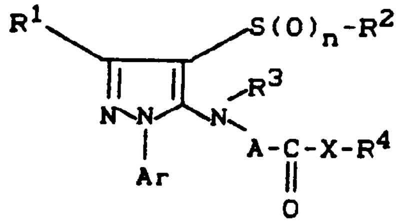

(I)

in welcher
R¹, R², R³, R⁴, A, X, Ar und n die oben angegebene Bedeutung haben,
wenn man 1-Arylpyrazole der Formel (II),

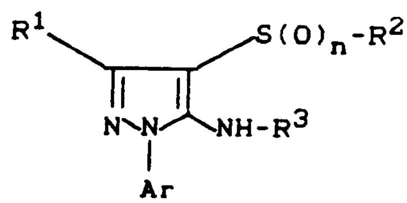

(II)

in welcher
R¹, R², R³, Ar und n die oben angegebene Bedeutung haben,
mit Alkylierungsmitteln der Formel (III),

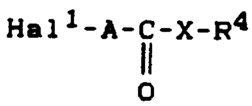

(III)

in welcher
Hal¹ für Chlor, Brom oder Jod steht und
R⁴, A und X die oben angegebene Bedeutung haben,
gegebenenfalls in Gegenwart eines Verdünnungsmittels und gegebenenfalls in Gegenwart eines Reaktionshilfsmittels umsetzt;
(b) man erhält substituierte 1-Arylpyrazole der Formel (Ib),

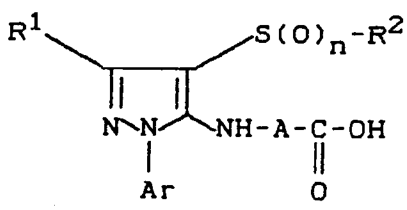

(Ib)

in welcher
R¹, R², A, Ar und n die oben angegebene Bedeutung haben,
wenn man substituierte 1-Arylpyrazole der Formel (Ia),

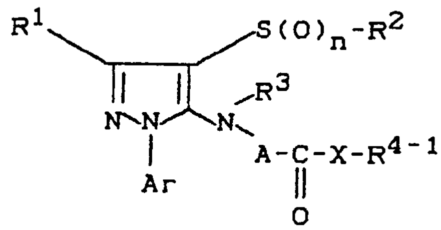

(Ia)

in welcher

$R^{4-1}$ für für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$, $R^2$, $R^3$, A, X, Ar und n die oben angegebene Bedeutung haben,

mit Säuren oder Basen als Katalysator in Gegenwart von Wasser sowie gegebenenfalls in Gegenwart eines Verdünnungsmittels verseift;

(c) man erhält substituierte 1-Arylpyrazole der Formel (Ic),

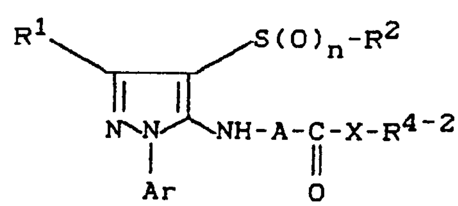

(Ic)

in welcher

$R^{4-2}$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und

$R^1$, $R^2$, A, X, Ar und n die oben angegebene Bedeutung haben,

wenn man substituierte 1-Arylpyrazole der Formel (Ib),

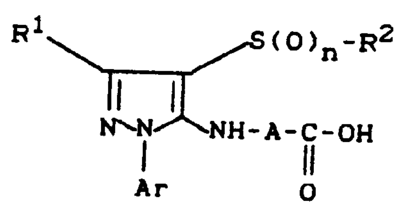

(Ib)

in welcher

$R^1$, $R^2$, A und n die oben angegebene Bedeutung haben,

mit Alkoholen, Thiolen oder Aminoverbindungen der Formel (IV),

$$R^{4-2}\text{-X-H} \qquad \text{(IV)}$$

in welcher

$R^{4-2}$ und X die oben angegebene Bedeutung haben,

in Gegenwart eines Acylierungskatalysators sowie gegebenenfalls in Gegenwart eines Verdünnungsmittels und gegebenenfalls in Gegenwart eines Reaktionshilfsmittels umsetzt;

(d) man erhält substituierte 1-Arylpyrazole der Formel (Id),

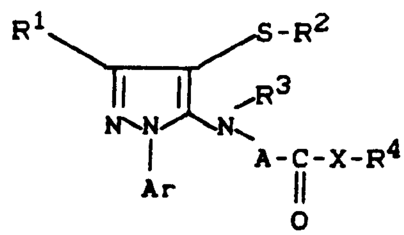

(Id)

in welcher

$R^1$, $R^2$, $R^3$, $R^4$, A, X und Ar die oben angegebene Bedeutung haben,

wenn man in 4-Stellung unsubstituierte 1-Arylpyrazole der Formel (V),

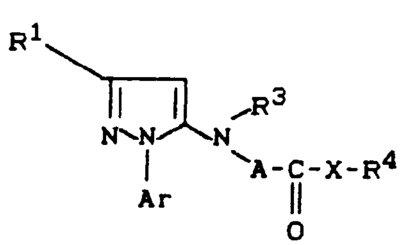

(V)

in welcher
$R^1$, $R^3$, $R^4$, A, X und Ar die oben angegebene Bedeutung haben,
mit Sulfenylhalogeniden der Formel (VI),

$R^2$-S-$Hal^2$ (VI)

in welcher
$Hal^2$ für Chlor oder Brom steht und
$R^2$ die oben angegebene Bedeutung hat,
gegebenenfalls in Gegenwart eines Verdünnungsmittels und gegebenenfalls in Gegenwart eines Reaktionshilfsmittels umsetzt;
(e) man erhält substituierte 1-Arylpyrazole der Formel (Ie),

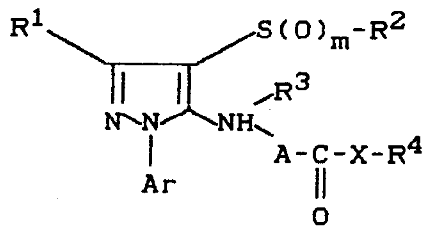

(Ie)

in welcher
$R^1$, $R^2$, $R^3$, $R^4$, A, X und Ar die oben angegebene Bedeutung haben und
m für eine Zahl 1 oder 2 steht,
wenn man substituierte 1-Arylpyrazole der Formel (Id),

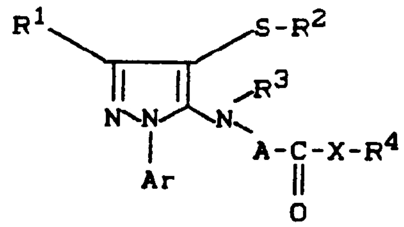

(Id)

in welcher
$R^1$, $R^2$, $R^3$, $R^4$, A, X und Ar die oben angegebene Bedeutung haben,
mit Oxidationsmitteln gegebenenfalls in Gegenwart eines Verdünnungsmittels und gegebenenfalls in Gegenwart eines Katalysators umsetzt;
(f) man erhält substituierte 1-Arylpyrazole der Formel (If),

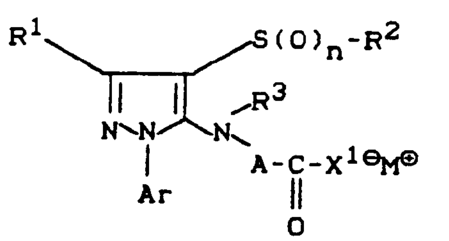

(If)

in welcher
$X^1$ für Sauerstoff oder Schwefel steht,
$M^{\oplus}$ für ein salzartig gebundenes Kation steht und
$R^1$, $R^2$, $R^3$, A, Ar und n die oben angegebene Bedeutung haben, wenn man substituierte 1-Arylpyrazole der Formel (Ig),

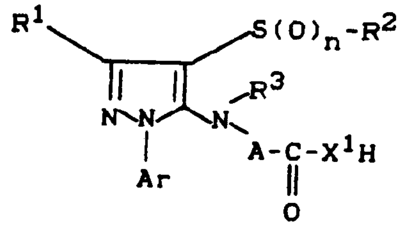

(Ig)

in welcher

$R^1$, $R^2$, $R^3$, A, $X^1$, Ar und n die oben angegebene Bedeutung haben,

mit anorganischen oder organischen Basen gegebenenfalls in Gegenwart eines Verdünnungsmittels umsetzt.

Schließlich wurde gefunden, daß die neuen substituierten 1-Arylpyrazole der allgemeinen Formel (I) gute Wirksamkeit gegen Schädlinge, insbesondere gegen pflanzenschädigende Blatt- und Bodeninsekten besitzen.

Überraschenderweise zeigen die erfindungsgemäßen substituierten 1-Arylpyrazole der allgemeinen Formel (I) eine deutlich bessere Wirksamkeit gegen Schädlinge als die aus dem Stand der Technik bekannten substituierten 1-Arylpyrazole wie beispielsweise die Verbindung 5-Amino-1-(2,6-dichlor-4-trifluormethylphenyl)-4-methylthiopyrazol oder die Verbindung 5-Amino-1-(2,6-dichlor-4-trifluormethylphenyl)-3-methyl-4-dichlorfluormethylthiopyrazol oder die Verbindung 1-(2,6-Dichlor-4-trifluormethylphenyl)-3-methyl-4-dichlorfluormethylsulfonyl-5-propionamidopyrazol oder die Verbindung 5-Amino-1-(2,6-dichlor-4-trifluormethylphenyl)-3-methyl-4-dichlorfluormethylsulfinyl-pyrazol oder die Verbindung 5-Amino-1-(2,6-dichlor-4-trifluormethylphenyl)-4-trifluormethylsulfinyl-pyrazol welche chemisch und wirkungsmäßig naheliegende Verbindungen sind.

Die erfindungsgemäßen substituierten 1-Arylpyrazole sind durch die Formel (I) allgemein definiert.

1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und außerdem für den Fall, daß X für Sauerstoff oder Schwefel steht, auch für ein Äquivalent eines Alkali-, Erdalkali-, Kupfer-, Zink-, Mangan-, Zinn-, Eisen-Cobalt- oder Nickelkations oder für ein gegebenenfalls einfach oder mehrfach, gleich oder verschieden substituiertes Ammonium-, Phosphonium- oder Sulfoniumkation steht, wobei als Substituenten infrage kommen: geradkettiges oder verzweigtes Alkyl mit 1 bis 18 Kohlenstoffatomen, Phenyl oder Benzyl,

A für einen zweifach verknüpften, geradkettigen oder verzweigten Alkylenrest mit 1 bis 12 Kohlenstoffatomen steht,

X für Sauerstoff, Schwefel oder für einen Rest

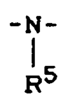

steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4

Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^6$ und,

n für eine Zahl 0, 1 oder 2 steht, wobei

$R^5$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen:

Bevorzugt sind Verbindungen der Formel (I), bei welchen

$R^1$ für Wasserstoff, Methyl, Ethyl, n- oder i-Propyl, n-, i-, s- oder t-Butyl steht,

$R^2$ für Methyl, Ethyl, n- oder i-Propyl, n-, i-, s- oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder Brompropyl steht,

$R^3$ für Wasserstoff, Acetyl, Propionyl oder Butyryl steht,

$R^4$ für Wasserstoff, für gegebenenfalls ein- bis dreifach, gleich oder verschieden durch Fluor, Chlor, Hydroxy, Methoxy, Ethoxy, Methylthio oder Ethylthio substituiertes Methyl, Ethyl, n- oder i-Propyl, n-, i-, s- oder t-Butyl oder n- oder i-Pentyl, für gegebenenfalls ein- bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein- bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, und außerdem für den Fall, daß X für Sauerstoff oder Schwefel steht, auch für ein Äquivalent eines Natrium-, Kalium-, Magnesium-, Calcium-, Barium-, Kupfer-, Zink-, Mangan-, Zinn-, Eisen-, Cobalt- oder Nickelkations oder für ein gegebenenfalls ein- bis vierfach, gleich oder verschieden durch Methyl, Ethyl, n-oder i-Propyl, n-, i-, s- oder t-Butyl, Benzyl oder Phenyl substituiertes Ammonium-, Phosphonium- oder Sulfoniumkation steht,

A für einen zweifach verknüpften, geradkettigen oder verzweigten Alkylenrest mit 1 bis 6 Kohlenstoffatomen steht,

X für Sauerstoff, Schwefel oder für einen Rest

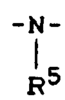

steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^6$ und

n für eine Zahl 0, 1 oder 2 steht, wobei

$R^5$ für Wasserstoff, für gegebenenfalls ein- bis dreifach, gleich oder verschieden durch Fluor, Chlor, Hydroxy, Methoxy, Ethoxy, Methylthio oder Ethylthio substituiertes Methyl, Ethyl, n- oder i-Propyl, n-, i-, s- oder t-Butyl oder n- oder i-Pentyl, für gegebenenfalls ein- bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein- bis fünffach, gleich

oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht,

$R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,

p für eine Zahl 0, 1 oder 2 steht.

Besonders bevorzugt sind Verbindungen der Formel (I), bei welchen

$R^1$ für Wasserstoff oder Methyl steht,

$R^2$ für Methyl, Ethyl, n- oder i-Propyl, Trifluormethyl, Dichlorfluormethyl oder Chlordifluormethyl steht,

$R^3$ für Wasserstoff, Acetyl oder Propionyl steht,

$R^4$ für Methyl, Ethyl, n- oder i-Propyl, n-, i-, s- oder t-Butyl, n- oder i-Pentyl, Allyl, Propenyl, n- oder i-Butenyl, n- oder i-Pentenyl, Propargyl, Propinyl, n- oder i-Butinyl, n- oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Propoxymethyl, Propoxyethyl, Butoxymethyl, Butoxyethyl, Methylthiomethyl, Methylthioethyl, Methylthiopropyl, Ethylthioethyl, Ethylthiopropyl oder Propylthioethyl steht, und außerdem für den Fall, daß X für Sauerstoff oder Schwefel auch für ein Natrium- oder Kaliumion oder für ein gegebenenfalls ein- bis vierfach, gleich oder verschieden durch Methyl, Ethyl, n- oder i-Propyl, n- Butyl oder Benzyl substituiertes Ammoniumion steht,

A für einen zweifach verknüpften, geradkettigen oder verzweigten Alkylenrest mit 1 bis 4 Kohlenstoffatomen steht,

X für Sauerstoff, Schwefel oder für

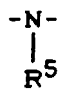

steht,

Ar für gegebenenfalls ein- bis fünffach gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl und

n für eine Zahl 0, 1 oder 2 steht, wobei

$R^5$ für Wasserstoff, Methyl, Ethyl, n- oder i-Propyl, n-, i-, s- oder t-Butyl, n- oder i-Pentyl, Allyl, Propenyl, n- oder i-Butenyl, n- oder i-Pentenyl, Propargyl, Propinyl, n- oder i-Butinyl, n- oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Methylthiomethyl Methylthioethyl, Methylthiopropyl, Ethylthioethyl oder Ethylthiopropyl steht.

Ganz besonders bevorzugt sind daneben auch Verbindungen der Formel (I), bei welchen

$R^1$ für Wasserstoff oder Methyl steht,

$R^2$ für Methyl, Ethyl, n- oder i-Propyl, Trifluormethyl, Dichlorfluormethyl oder Chlordifluormethyl steht,

$R^3$ für Wasserstoff steht,

$R^4$ für Wasserstoff oder für ein Äquivalent eines Natrium-, Kalium-, Calcium-, Magnesium-, Barium-, Kupfer-, Zink-, Mangan-, Zinn-, Eisen-, Cobalt- oder Nickelkations steht oder für ein gegebenenfalls ein- bis vierfach, gleich oder verschieden durch Methyl, Ethyl, n- oder i-Propyl, n-, i- oder s-Butyl, Benzyl oder Phenyl substituiertes Ammoniumkation steht,

A für einen zweifach verknüpften, geradkettigen oder verzweigten Alkylenrest mit 1 bis 4 Kohlenstoffatomen steht,

X für Sauerstoff steht,

Ar für gegebenenfalls ein- bis fünffach, gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethylsulfinyl oder Trifluormethylsulfonyl und

n für eine Zahl 0, 1 oder 2 steht.

Insbesondere bevorzugt sind Verbindungen der Formel (I), bei welchen

$R^1$ für Wasserstoff oder Methyl steht,

$R^2$ für Trifluormethyl, Dichlorfluormethyl oder Chlordifluormethyl steht,

$R^3$ für Wasserstoff, Acetyl oder Propionyl steht,

$R^4$ für Wasserstoff, Methyl, Ethyl, n- oder i-Propyl, n-, i-, s- oder t-Butyl oder Benzyl steht,

A für einen Rest der Formel $-CH_2-$; $-CH_2-CH_2-$; $-CH_2-CH_2-CH_2-$ oder

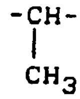

steht,

X für Sauerstoff oder für einen Rest

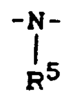

steht,

Ar für gegebenenfalls ein- bis fünffach, gleich oder verschieden durch Fluor, Chlor, Brom, Trifluormethyl oder Trifluormethoxy substituiertes Phenyl steht und

n für eine Zahl 0, 1 oder 2 steht, wobei

$R^5$ für Methyl, Ethyl, n- oder i-Propyl, n-, i-, s- oder t-Butyl oder für einen Rest $-SO_2-R^6$ steht und

$R^6$ für Methyl, Trifluormethyl, Phenyl oder p-Tolyl steht.

Verwendet man beispielsweise 5-Acetamido-3-methyl-1-(2,6-dichlor-4-trifluormethylphenyl)-4-trifluormethylthio-pyrazol und Bromessigsäuremethylester als Ausgangsstoffe, so läßt sich der Reaktionsablauf des erfindungsgemäßen Verfahrens (a) durch das folgende Formelschema darstellen:

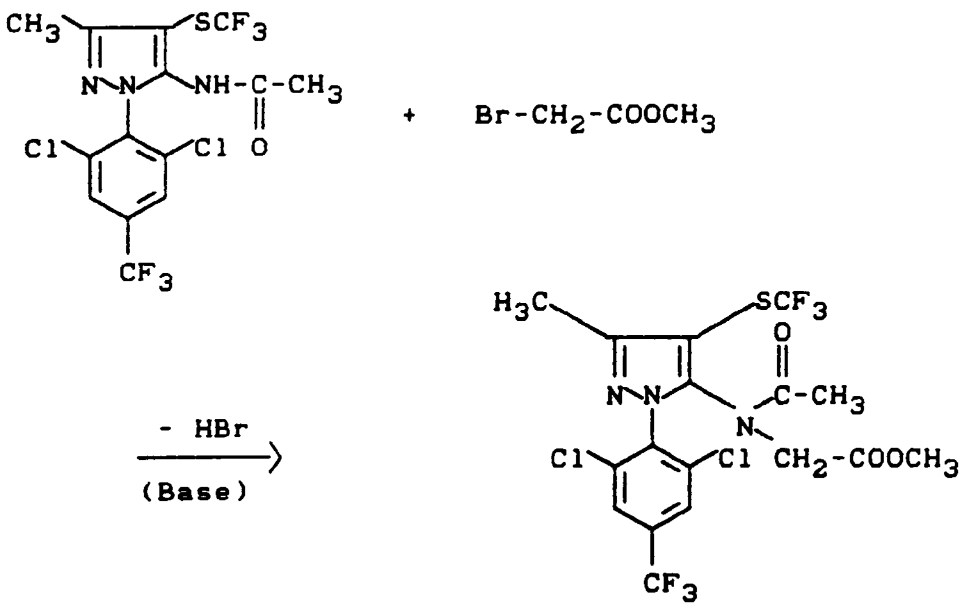


Verwendet man beispielsweise 5-[N-methoxycarbonylmethyl)-acetamido]-3-methyl-1-(2,6-dichlor-4-trifluormethylphenyl)-4-trifluormethylthio-pyrazol als Ausgangsverbindung, so läßt sich der Reaktionsablauf des erfindungsgemäßen Verfahrens (b) durch das folgende Formelschema darstellen:

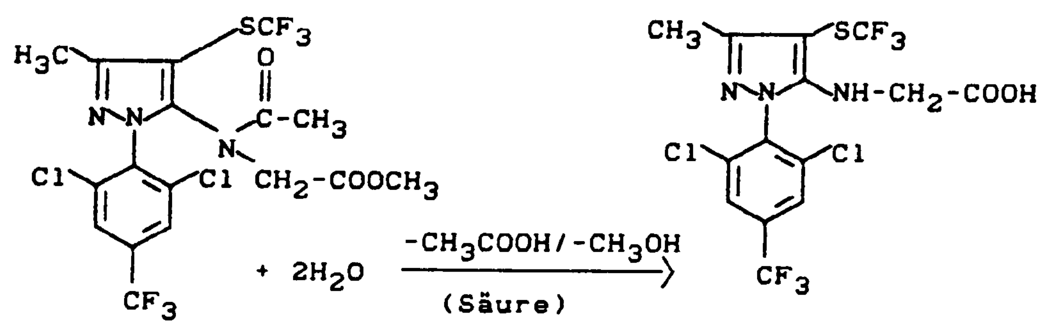


Verwendet man beispielsweise N-[4-Methylthio-1-(2,4,6-trichlorphenyl)-5-pyrazolyl]-glycin und 2-Methoxyethanol als Ausgangsstoffe, so läßt sich der Reaktionsablauf des erfindungsgemäßen Verfahren (c) durch das folgende Formelschema darstellen:

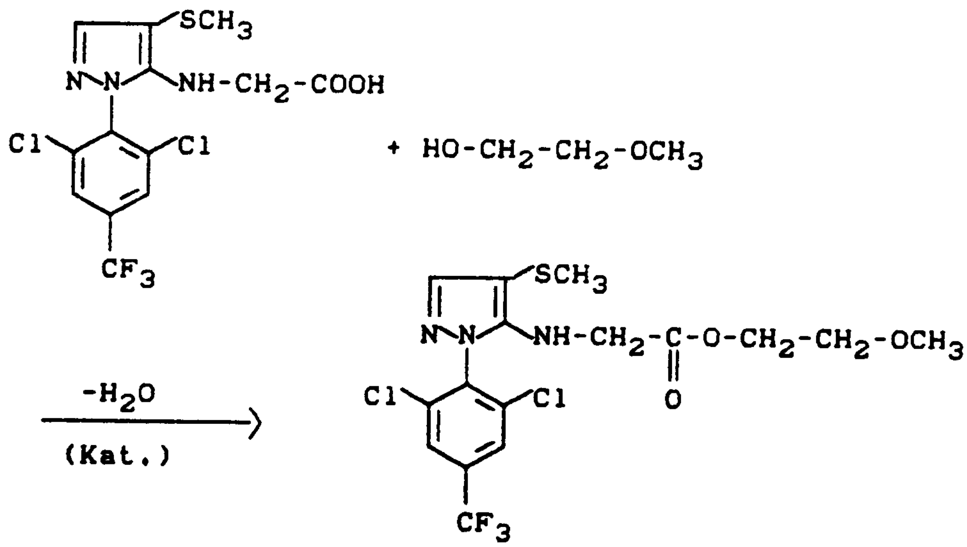


Verwendet man beispielsweise N-[1-(2,6-Dichlor-4-trifluormethylphenyl)-5-pyrazolyl]-glycin und Dichlorfluormethansulfenylchlorid als Ausgangsstoffe, so läßt sich der Reaktionsablauf des erfindungsgemäßen Verfahrens (d) durch das folgende Formelschema darstellen:

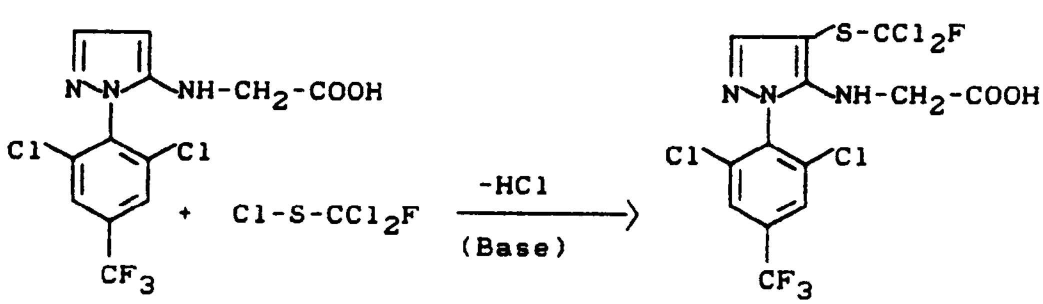


Verwendet man beispielsweise 3-Methyl-4-methyl-thio)-1-(2,4-dichlorphenyl)-5-ethoxycarbonyl-methylaminopyrazol als Ausgangsverbindung und 3-Chlorperbenzoesäure als Oxidationsmittel, so läßt sich der Reaktionsablauf des erfindungsgemäßen Verfahrens (e) durch das folgende Formelschema darstellen:

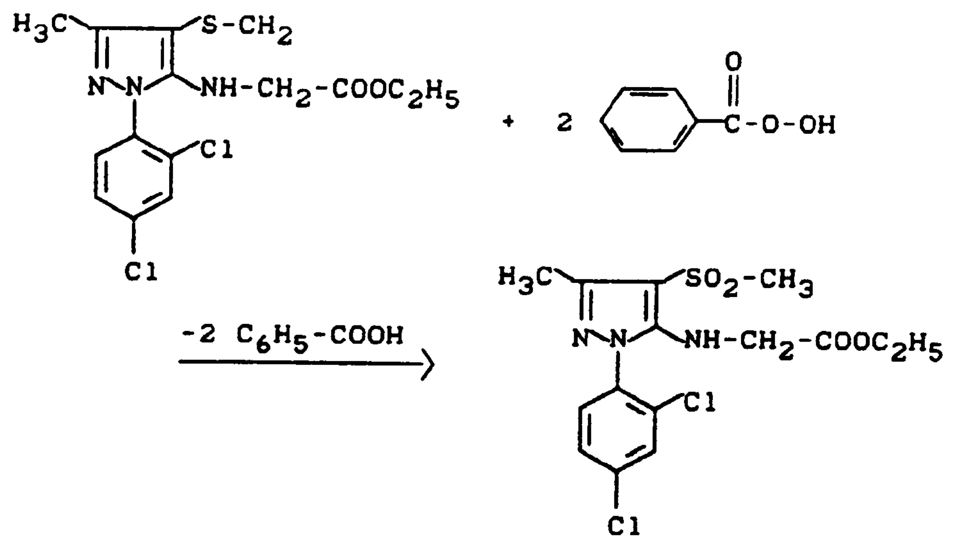


Verwendet man beispielsweise N-[1-(2,6-Dichlor-4-trifluormethylsulfonyl-phenyl)-3-methyl-4-trifluormethylsulfonyl-5-pyrazolyl]-glycin und Triethylamin als Ausgangsstoffe, so läßt sich der Reaktionsablauf des erfindungsgemäßen Verfahrens (f) durch das folgende Formelschema darstellen:

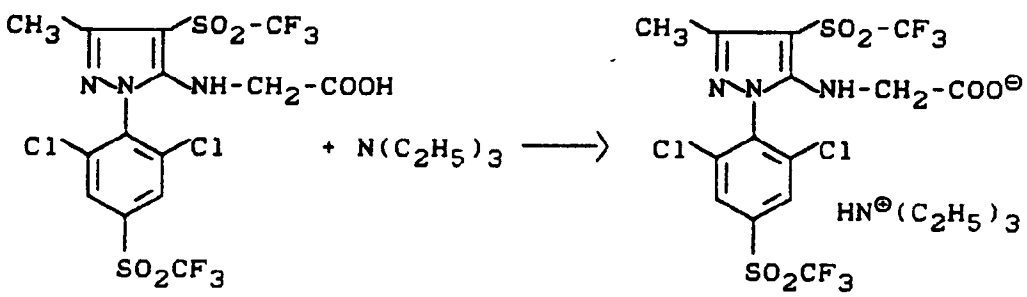

Die zur Durchführung des erfindungsgemäßen Verfahrens (a) als Ausgangsstoffe benötigten 1-Arylpyrazole sind durch die Formel (II) allgemein definiert. In dieser Formel (II) stehen $R^1$, $R^2$, $R^3$, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die 1-Arylpyrazole der Formel (II) sind bekannt (vgl. z.B. EP-A 201 852).

Die zur Durchführung des erfindungsgemäßen Verfahrens (a) weiterhin als Ausgangsstoffe benötigten Alkylierungsmittel sind durch die Formel (III) allgemein definiert. In dieser Formel (III) stehen $R^4$, A und X vorzugsweise für diejenigen Reste, die bereits im Zusammenhang mit der Beschreibung der erfindungsgemäßen Stoffe der Formel (I) als bevorzugt für diese Substituenten genannt wurden.

$Hal^1$ steht für Chlor, Brom oder Iod, vorzugsweise für Brom.

Die Alkylierungsmittel der Formel (II) sind allgemein bekannte Verbindungen der organischen Chemie oder erhältlich in Analogie zu allgemeinen bekannten Verfahren.

Die zur Durchführung des erfindungsgemäßen Verfahrens (b) als Ausgangsstoffe benötigten substituierten 1-Arylpyrazole sind durch die Formel (Ia) allgemein definiert. In dieser Formel (Ia) stehen $R^1$, $R^2$, $R^3$, A, X, Ar und n vorzugsweise für diejenigen Reste, die bereits im Zusammenhang mit der Beschreibung der erfindungsgemäßen Stoffe der Formel (I) als bevorzugt für diesen Substituenten genannt wurden.

$R^{4-1}$ steht vorzugsweise für diejenigen Reste, die bereits im Zusammenhang mit der Beschreibung der erfindungsgemäßen Stoffe der Formel (I) als bevorzugt für den Substituenten $R^4$ genannt wurden, mit Ausnahme des Wasserstoffrestes und der salzartig gebundenen Kationen.

Die substituierten 1-Arylpyrazole der Formel (Ia) sind erfindungsgemäßen Verbindungen und erhältlich mit Hilfe der erfindungsgemäßen Verfahren (a), (c), (d) oder (e).

Die zur Durchführung des erfindungsgemäßen Verfahrens (c) als Ausgangsstoffe benötigten substituierten 1-Arylpyrazole sind durch die Formel (Ib) allgemein definiert. In dieser Formel (Ib) stehen $R^1$, $R^2$, A, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten 1-Arylpyrazole der Formel (Ib) sind erfindungsgemäße Verbindungen und erhältlich mit Hilfe der erfindungsgemäßen Verfahren (a), (b), (d) oder (e).

Die zur Durchführung des erfindungsgemäßen Verfahrens (c) weiterhin als Ausgangsstoffe benötigten Alkohole, Thiole oder Aminoverbindungen sind durch die Formel (IV) allgemein definiert. In dieser Formel (IV) steht X vorzugsweise für diejenigen Reste, die bereits im Zusammenhang mit der Beschreibung der erfindungsgemäßen Stoffe der Formel (I) als bevorzugt für diese Substituenten genannt wurden.

$R^{4-2}$ steht vorzugsweise für diejenigen Rest, die bereits im Zusammenhang mit der Beschreibung der erfindungsgemäßen Stoffe der Formel (I) als bevorzugt für den Substituenten $R^4$ genannt wurden, mit Ausnahme der salzartig gebundenen Kationen.

Die Alkohole, Thiole oder Aminoverbindungen der Formel (IV) sind allgemein bekannte Verbindungen der organischen Chemie.

Die zur Durchführung des erfindungsgemäßen Verfahrens (d) als Ausgangsstoffe benötigten in 4-Stellung unsubstituierten 1-Arylpyrazole sind durch die Formel (V) allgemein definiert. In dieser Formel (V) stehen $R^1$, $R^3$, $R^4$, A, X und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die in 4-Stellung unsubstituierten 1-Arylpyrazole der Formel (V) sind noch nicht bekannt. Man erhält sie jedoch in Analogie zu bekannten Verfahren, beispielsweise wenn man 1-Arylpyrazole der Formel (VII),

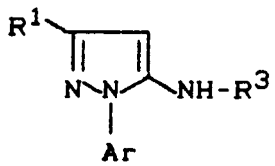

(VIII)

in welcher

$R^1$, $R^3$ und Ar die oben angegebene Bedeutung haben,

in Analogie zur Durchführung des erfindungsgemäßen Verfahrens (a) mit Alkylierungsmitteln der Formel (III),

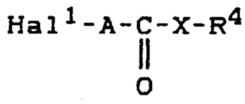

(III)

in welcher

A, X und $R^4$ die oben angegebene Bedeutung haben und

$Hal^1$ für Chlor, Brom, Jod, vorzugsweise für Brom steht,

gegebenenfalls in Gegenwart eines Verdünnungsmittels wie beispielsweise Acetonitril oder Tetrahydrofuran und gegebenenfalls in Gegenwart eines Reaktionshilfsmittels wie beispielsweise Kaliumcarbonat oder Natriumhydrid bei Temperaturen zwischen - 30 °C und + 120 °C umsetzt oder alternativ auch wenn man 1-Arylpyrazole der Formel (VIII),

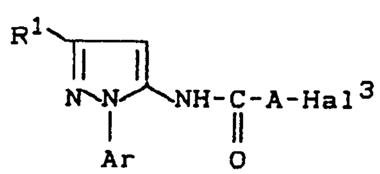

(VIII)

in welcher

$R^1$, A und Ar die oben angegebene Bedeutung haben und

$Hal^3$ für Chlor, Brom, Jod insbesondere für Chlor oder Brom steht,

zunächst in einer ersten Stufe mit einer Base, wie beispielsweise Ammoniak, gegebenenfalls in Gegenwart eines Verdünnungsmittels wie beispielsweise Ethanol intramolekular cyclisiert und anschließend in einer zweiten Stufe die so erhältlichen N-Pyrazolyllactame der Formel (IX),

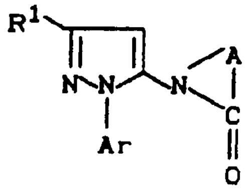

(IX)

in welcher

$R^1$, A und Ar die oben angegebene Bedeutung haben,

mit wässrigen Säuren wie beispielsweise Bromwasserstoffsäure bei Temperaturen zuwischen 20 °C und 150 °C wieder Spaltet zu den in 4-Stelltung unsubstituierten 1-Arylpyrazolen der Formel (Va),

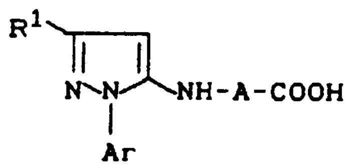

(Va)

in welcher

$R^1$, A und Ar die oben angegebene Bedeutung haben (vgl. auch die Herstellungsbeispiele).

1-Arylpyrazole der Formeln (VII) und (VIII) sind bekannt (vgl. z.B. EP-A 154 115; EP-A 201 852).

Die zur Durchführung des erfindungsgemäßen Verfahrens (d) weiterhin als Ausgangsstoffe benötigten Sulfenylhalogenide sind durch die Formel (VI)) allgemein definiert. In dieser Formel (IV)) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^2$ steht für Chlor oder Brom.

Die Sulfenylhalogenide der Formel (VI) sind allgemein bekannte Verbindungen der organischen Chemie.

Die zur Durchführung des erfindungsgemäßen Verfahrens (e) als Ausgangsstoffe benötigten substituierten 1-Arylpyrazole sind durch die Formel (Id) allgemein definiert. In dieser Formel (Id) stehen $R^1$, $R^2$, $R^3$, $R^4$, A, X und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten 1-Arylpyrazole der Formel (Id) sind erfindungsgemäße Verbindungen und erhältlich mit Hilfe der erfindungsgemäßen Verfahren (a), (b), (c), (d) und (f).

Die zur Durchführung des erfindungsgemäßen Verfahrens (f) als Ausgangsstoffe benötigten substituierten 1-Arylpyrazole sind durch die Formel (Ig) allgemein definiert. In dieser Formel (Ig) stehen $R^1$, $R^2$, $R^3$, A, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

$X^1$ steht für Sauerstoff, Schwefel oder für einen Rest der Formel

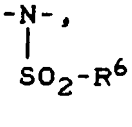

wobei $R^6$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die substituierten 1-Arylpyrazole der Formel (Ig) sind erfindungsgemäße Verbindungen und erhältlich mit Hilfe der erfindungsgemäßen Verfahren (a), (b), (d) und (e).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Reaktionshilfsmittel zur Durchführung des erfindungsgemäßen Verfahrens (a) kommen alle üblicherweise verwendbaren anorganischen und organischen Basen infrage. Vorzugsweise verwendet man Alkalimetallhydride, -hydroxide, -amide, -alkoholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (a) setzt man pro Mol 1-Aryl-pyrazol der Formel (II) im allgemeinen 1.0 bis 20.0 Mol, vorzugsweise 1.0 bis 15.0 Mol an Alkylierungsmittel der Formel (III) und gegebenenfalls 1.0 bis 3.0 Mol, vorzugsweise 1.0 bis 2.0 Mol an Reaktionshilfsmittel sowie gegebenenfalls 0,01 bis 1,0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen anorganische oder organische polare Lösungsmittel in Frage. Vorzugsweise verwendet man Alkohole, wie beispielsweise Methanol, Ethanol oder Propanol oder deren Gemische mit Wasser.

Als Katalysatoren zur Durchführung des erfindungsgemäßen Verfahrens (b) kommen vorzugsweise Säuren, insbesondere Chlorwasserstoffsäure oder Schwefelsäure oder Basen, in besondere Natriumhydroxid, Natriumhydrid oder Kalium-t-butylat infrage.

Die Reaktionstemperaturen können bei der Durchführung des erfindungsgemäßen Verfahrens (b) in einem größeren Bereich var iert werden. Im allgemeinen arbeitet man zwischen +20°C und +150°C, vorzugsweise zwischen +50°C und +120°C.

Zur Durchführung des erfindungsgemäßen Verfahrens (b) setzt ma pro Mol an substituiertem 1-Arylpyrazol der Formel (Ia) im allgemeinen 1,0 bis 20,0 Mol, vorzugsweise 1,0 bis 10,0 Mol an Katalysatorsäure oder -base ein und erwärmt für mehrere Stunden auf die erforderliche Reaktionstemperatur. Die Aufarbeitung, Isolierung und Reinigung der Reaktionsprodukte der Formel (Ib) erfolgt nach üblichen Methoden.

Als Verdünnungsmittel zur Durchführung des erfindungsgemäßen Verfahrens (c) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder diethylether, Ketone wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester wie Essigsäureethylester, oder Sulfoxide, wie Dimethylsulfoxid.

Verwendet man als Reaktionspartner der Formel (IV) Alkohole, Aminoverbindungen oder Thiole in flüssiger Form, so ist es auch möglich, diese in einem entsprechenden Überschuß gleichzeitig als Verdünnungsmittel einzusetzen.

Das erfindungsgemäße Verfahren (c) wird vorzugsweise in Gegenwart eines geeigneten Acylierungskatalysators durchgeführt. Als solche kommen prinzipiell alle üblichen für Veresterung und Amidierungen verwendbaren Reaktionshilfsmittel in Frage. Beispielhaft genannt seien Säurehalogenidbildner wie Thionylchlorid, Phosphortrichlorid, Phosphorpentachlorid, Phosphoroxychlorid, oder Aktivesterkomponenten wie N-Hydroxy-Succinimid, Anhydridbildner wie Chlorameisensäure-4-nitrophenylester oder übliche Kondensationsmittel wie konzentrierte Schwefelsäure, Dicyclohexylcarbodiimid (DCC), Triphenylphosphin im Gemisch mit Tetrachlorkohlenstoff, N,N'-Carbonyl-diimidazol oder N-Ethoxycarbonyl-2-ethoxy-dihydrochinolin (EEDQ).

Das erfindungsgemäße Verfahren (c) kann außerdem gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide wie Natriumhydroxdid oder Kaliumhydroxid, Alkalimetallcarbonate wie Natriumcarbonat, Kaliumcarbonat oder Natriumhydrogencarbonat, sowie tertiäre Amine wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Auch ein entsprechender Überschuß eines gleichzeitig als Reaktionspartner der Formel (IV) verwendeten Amins kann gegebenenfalls als Reaktionshilfsmittel dienen.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und +100°C.

Zur Durchführung des erfindungsgemäßen Verfahrens (c) setzt man pro Mol an substituierten 1-Arylpyrazol der Formel (Ib) im allgemeinen 1,0 bis 20,0 Mol, vorzugsweise 1,0 bis 10,0 Mol an Alkohol, Aminoverbindung oder Thiol der Formel (IV), 1,0 bis 5,0 Mol, vorzugsweise 1.0 bis 2,0 Mol an Acylierungskatalysator und gegebenenfalls 1,0 bis 2,0 Mol an Reaktionshilfsmittel ein.

In den meisten Fällen ist es vorteilhaft zunächst aus dem substituierten 1-Arylpyrazol der Formel (Ib) und dem Acylierungskatalysator nach üblichen Verfahren einen aktivierten Komplex (Säurehalogenid, Aktivester, gemischtes Säureanhydrid etc.) herzustellen, welcher gegebenenfalls isoliert werden kann und entweder in einem getrennten Reaktionsschritt oder im Eintopfverfahren mit dem Alkohol, Amin oder Thiol der Formel (IV) umgesetzt wird. Die Zugabe des Reaktionshilfsmittels kann dabei in Abhängigkeit von der Art des verwendeten Acylierungskatalysators entweder in der 1.Stufe zur Bildung des aktivierten Komplexes oder in der 2.Stufe zur Umsetzung desselben nützlich sein. Die Reaktionsdurchführung, Aufarbeitung und Isolierung der Reaktionsprodukte der Formel (Ic) erfolgt nach allgemein üblichen Verfahren.

Als Verdünnungsmittel zur Durchführung des erfindungsgemäßen Verfahrens (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren wie beispielsweise Essigsäure.

Das erfindungsgemäße Verfahren (d) kann gegebenenfalls in Gegenwart eines Reaktionshilfsmittels durchgeführt werden. Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan(DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen -20 °C und +120 °C, vorzugsweise bei Temperaturen zwischen 0 °C und +50 °C.

Zur Durchführung des erfindungsgemäßen Verfahrens (d) setzt man pro Mol in an 4-Stellung unsubstituiertem 1-Arylpyrazol der Formel (V) im allgemeinen 1.0 bis 2.5 Mol, vorzugsweise 1.0 bis 1.5 Mol an Sulfenylhalogenid der Formel (VI) und gegebenenfalls 1.0 bis 2.5 Mol, vorzugsweise 1.0 bis 1.5 Mol an Reaktionshilfsmittels ein. Die Reaktionsführung, Aufarbeitung und Isolierung der Reaktionsprodukte der Formel (Id) erfolgt nach allgemein üblichen Verfahren.

Als Oxidationsmittel zur Durchführung des erfindungsgemäßen Verfahrens (e) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (e) kommen ebenfalls inerte organische Lösungsmittel in Frage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (e) kann gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (e) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren in Frage. Beispielhaft genannt sei in diesem Zusammenhang Ammoniummolybdat.

Die Reaktionstemperaturen können bei der Durchführung des erfindungsgemäßen Verfahrens (e) in einem größeren Bereich variiert werden Im allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (f) setzt man pro Mol an substituiertem 1-Arylpyrazol der Formel (Id) im allgemeinen 0.8 bis 1.2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an substituiertem 1-Arylpyrazol der Formel (Id) im allgemeinen 1.8 bis 3.0 Mol, vorzugsweise doppelt molare Mengen an Oxidationsmittel ein. Die Reaktionsführung, Aufarbeitung und Isolierung der Endprodukte der Formel (Ie) erfolgt nach üblichen Verfahren.

Das erfindungsgemäße Verfahren (f) wird in Gegenwart einer anorganischen oder organischen Base durchgeführt. Als solche verwendet man Hydroxide, Oxide oder Carbonate von Alkali- oder Erdalkalimetallen oder geeignet substituierte Amine, in Abhängigkeit von der Art des gewünschten Gegenions $M^{\oplus}$ in den Verbindungen der Formel (If).

Als Verdünnungsmittel zur Durchführung des erfindungsgemäßen Verfahrens (f) kommen organische oder wässrige Lösungsmittel oder organisch-wässrige Lösungsmittelgemische in Frage. Vorzugsweise verwendet man Alkohole wie Methanol, Ethanol oder Propanol oder deren Gemische mit Wasser sowie reines Wasser als Verdünnungsmittel.

Die Reaktionstemperaturen können bei der Durchführung des erfindungsgemäßen Verfahrens (f) in einem größeren Bereich variiert werden. Im allgemeinen arbeitet man bei Temperaturen zwischen -20°C und +120°C, vorzugsweise bei Temperaturen zwischen 0°C und 80°C.

Zur Durchführung des erfindungsgemäßen Verfahrens (f) setzt man pro Mol an substituiertem 1-Arylpyrazol der Formel (Ig) im allgemeinen 1,0 bis 20,0 Mol, vorzugsweise 1,0 bis 10,0 Mol an Base ein. Die Calcium-, Barium-, Magnesium-, Mangan-, Kupfer-, Nickel-, Zinn-, Eisen- und Cobaltsalze erhält man auch aus den Natriumsalzen durch Behandeln mit einem entsprechenden anorganischen Metallsalz, z.B. Calciumchlorid, Bariumchlorid, Kupfersulfat, Nickelchlorid oder Cobaltnitrat. Die Aufarbeitung und Isolierung der Salze der Formel (If) erfolgt nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus,

Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme, sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide Wirksamkeit aus.

Sie lassen sich mit besonders gutem Erfolg zur Bekämpfung von pflanzenschädigenden Insekten, wie beispielsweise gegen die Raupen des Eulenfalters (Spodoptera frugiperda) oder gegen die Larven der Meerettichblattkäfer (Phaedon cochleariae) oder gegen die grüne Pfirsichblattlaus (Mycus persicae) ebenso wie zur Bekämpfung von Bodeninsekten, wie beispielsweise gegen die Maden der Zwiebelfliege (Phorbia antiqua) oder von Diabrotica balteata-Larven im Boden einsetzen. Dabei zeigen die erfindungsgemäßen Wirkstoffe auch systemische Eigenschaften und eignen sich daher auch besonders gut als Saatgutbehandlungsmittel.

Darüber hinaus lassen sich die erfindungsgemäßen Wirkstoffe mit besonders gutem Erfolg zur Bekämpfung von parasitisch lebenden Warmblüterschädlingen, wie beispielsweise gegen Rinderzecken (Boophilus microplus) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden:

Herstellungsbeispiele

Beispiel 1

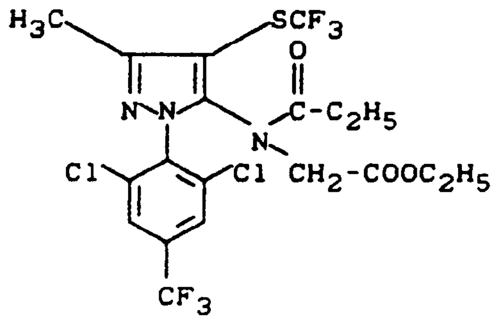

(Verfahren a)

Zu einer Mischung aus 9,3 g (0,02 Mol) 1-(2,6-Dichlor-4-trifluormethylphenyl)-3-methyl-4-trifluormethylthio-5-propionamidopyrazol (vgl. z.B. EP 201 852) und 0,87 g (0,03 Mol) Natriumhydrid (80 %ig in Paraffin) in 90 ml absolutem Tetrahydrofuran gibt man bei Raumtemperatur tropfenweise unter Rühren 3,4 ml (0,03 Mol) Bromessigsäureethylester, rührt nach beendeter Zugabe weitere 3 Stunden bei Raumtemperatur, gibt dann 100 ml gesättigte wässrige Ammoniumchloridlösung zu, extrahiert mehrfach mit Dichlormethan, wäscht die vereinigten organischen Extrakte mit gesättigter wässriger Natriumchloridlösung, trocknet über Natriumsulfat und entfernt das Lösungsmittel im Vakuum.

Man erhält 11,1 g (100 % der Theorie) an N-Propionyl-N-[1-(2,6-dichlor-4-trifluormethylphenyl)-3-methyl-4-trifluormethylthio-5-pyrazolyl]-glycinethylester vom Schmelzpunkt 73 °C bis 74 °C.

Beispiel 2

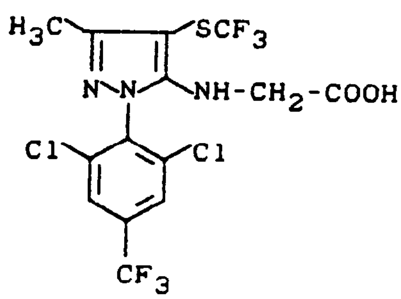

(Verfahren b)

6 g (0,011 Mol) N-Propionyl-N-[1-(2,6-dichlor-4-trifluormethylphenyl)-3-methyl-4-trifluormethylthio-5-pyrazolyl]-glycinethylester werden in 25 ml 80 %iger Schwefelsäure ca. 8 Stunden bei 50 °C gerührt. Anschließend wird die Reaktionsmischung in 200 ml Eiswasser gegeben, ausgefallenes Produkt abfiltriert, in 2normaler Natronlauge gelöst, mit Dichlormethan gewaschen, durch Ansäuern unter Eiskühlung wieder ausgefällt abgesaugt mit Wasser gewaschen und getrocknet.

Man erhält 4,3 g (84 % der Theorie) an N-[1-(2,6-Dichlor-4-trifluormethylphenyl)-3-methyl-4-trifluormethylthio-5-pyrazolyl]-glycin vom Schmelzpunkt 168-170 °C.

Beispiel 3

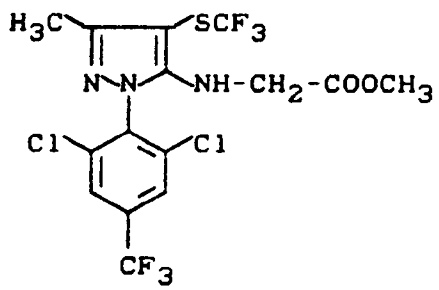

(Verfahren c)

Eine Lösung aus 3 g (0,0062 Mol) N-[1-(2,6-Dichlor-4-trifluormethylphenyl)-3-methyl-4-trifluormethylthio-5-pyrazolyl]-glycin und 0,9 ml konzentrierter Schwefelsäure in 30 ml absolutem Methanol wird 4 Stunden bei 20 °C gerührt, anschließend im Vakuum eingeengt, der Rückstand in Ether aufgenommen, mit gesättigter wässriger Natriumhydrogencarbonatlösung gewaschen, über Natriumsulfat getrocknet und im Vakuum eingeengt.

Man erhält 2,3 g (77 % der Theorie) an N-[1-(2,6-Dichlor-4-trifluormethylphenyl)-3-methyl-4-trifluormethylthio-5-pyrazolyl]-glycinmethylester als Öl.
$^1$H-NMR ($CDCl_3$/Tetramethylsilan): δ = 7,77 (s, 2H, Aryl) ppm.

Beispiel 4

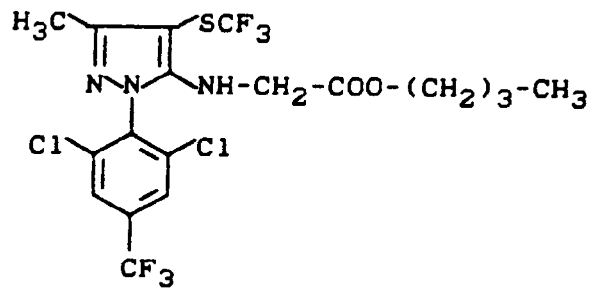

(Verfahren c)

Eine Mischung aus 4,7 g (0,01 Mol) N-[1-(2,6-Dichlor-4-trifluormethylphenyl)-3-methyl-4-trifluormethylthio-5-pyrazolyl]-glycin, 2,1 g (0,01 Mol) Phosphorpentachlorid und 100 ml absolutem Ether wird 15 Minuten auf Rückflußtemperatur erhitzt, anschließend im Vakuum eingeengt, der Rückstand in 50 ml n-Butanol aufgenommen und 16 Stunden bei Raumtemperatur gerührt. Zur Aufarbeitung wird im Vakuum eingeengt, der Rückstand in Dichlormethan aufgenommen, mit Wasser gewaschen, über Natriumsulfat getrocknet und im Vakuum vom Lösungsmittel befreit.

Man erhält 3,5 g (67 % der Theorie) an N-[1-(2,6-Dichlor-4-trifluormethylphenyl)-3-methyl-4-trifluormethylthio-5-pyrazolyl]-glycin-n-butylester als Öl.
$^1$H-NMR ($CDCl_3$/Tetramethylsilan): δ = 7,75 ppm (s, 2H, Aryl).

Beispiel 5

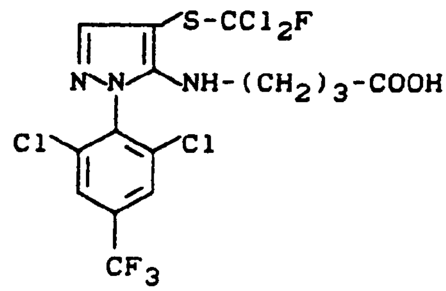

(Verfahren d)

Zu 10 g (0,0262 Mol) N-[1-(2,6-Dichlor-4-trifluormethylphenyl)-5-pyrazolyl]-4-aminobuttersäure in 40 ml Eisessig gibt man bei 15 °C tropfenweise unter Rühren 3,3 ml (0,0315 Mol) Dichlorfluormethansulfenylchlorid zu, rührt anschließend 5 Stunden bei Raumtemperatur, gibt dann die Reaktionsmischung in Eiswasser, saugt ausgefallenes Produkt ab, wäscht mit Wasser nach und trocknet im Vakuum bei 50 °C.

Man erhält 12,0 g (89 % der Theorie) an N-[1-(2,6-Dichlor-4-trifluormethylphenyl)-4-dichlorfluormethansulfenyl-5-pyrazolyl]-4-aminobuttersäure vom Schmelzpunkt 99 °C bis 101 °C.

Herstellung der Ausgangsverbindung

Beispiel V-1

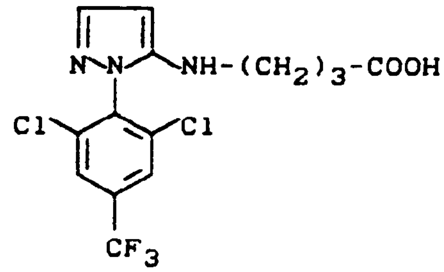

61,5 g (0,169 Mol) 1-[1-(2,6-Dichlor-4-trifluormethylphenyl)-5-pyrazolyl]-pyrrolidin-2-on werden in 250 ml 48prozentiger Bromwasserstoffsäure 24 Stunden unter Rühren auf Rückflußtemperatur erhitzt. Zur Aufarbeitung wird im Vakuum eingeeng, der Rückstand in Wasser suspendiert mit 4prozentiger wässriger Natronlauge neutralisiert, ausgefallener Niederschlag abgesaugt, gewaschen und getrocknet, in n-Hexan aufgenommen, verrührt, erneut abgesaugt und im Vakuum bei 50 °C getrocknet.

Man erhält 39,4 g (61 % der Theorie) an N-[1-(2,6-Dichlor-4-trifluormethylphenyl)-5-pyrazolyl]-4-aminobuttersäure vom Schmelzpunkt 160-162 °C.

Beispiel IX-1

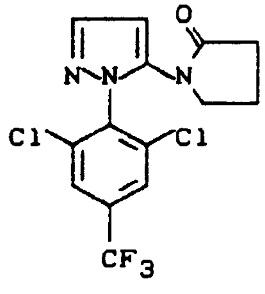

Zu 67,7 g (0,169 Mol) 5-(4-Chlorbutanoyl)-amino-1-(2,6-dichlor-4-trifluormethylphenyl)-pyrazol (vgl. z.B. EP 154 115) in 120 ml Ethanol gibt man 30 ml konzentriertes Ammoniakwasser, rührt 24 Stunden bei Raumtemperatur, engt im Vakuum ein und setzt so erhältliches Rohprodukt direkt weiter um zu Beispiel V-1.

Man erhält 61,5 g (98 % der Theorie) an 1-[1-(2,6-Dichlor-4-trifluormethylphenyl)-5-pyrazolyl]-pyrrolidin-2-on vom Schmelzpunkt 130-131 °C

Beispiel VIII-1

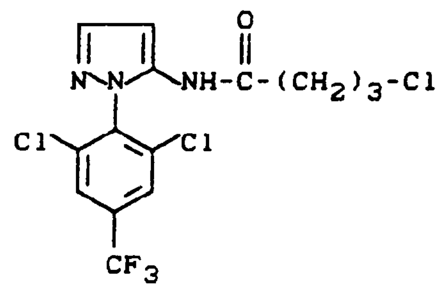

Zu 50 g (0,169 Mol) 5-Amino-1-(2,6-dichlor-4-trifluormethylphenyl)-pyrazol (vgl. EP 154 115) in 250 ml Acetonitril gibt man zunächst 15 ml (0,186 Mol) wasserfreies Pyridin und anschließend bei Raumtemperatur unter Rühren tropfenweise 20,3 ml (0,178 Mol) 4-Chlorbutanoylchlorid, wobei die Temperatur der Reaktionsmischung auf 30 °C ansteigt. Nach beendeter Zugabe rührt man eine weitere Stunde bei Raumtemperatur, gibt dann die Reaktionsmischung unter Rühren in 1 000 ml Wasser, saugt ausgefallenes Produkt ab und trocknet im Vakuum bei 50 °C.

Man erhält 67,7 g (100 % der Theorie) an 5-(4-Chlorbutanoylamino)-1-(2,6-dichlor-4-trifluormethylphenyl)-pyrazol vom Schmelzpunkt 103 bis 104 °C.

In entsprechender Weise und gemäß den allgemeinen Angaben zur Herstellung erhält man die folgende substituierten 1-Arylpyrazole der allgemeinen Formel (I):

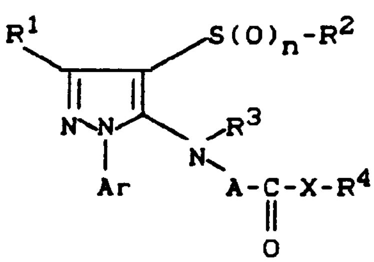

(I)

| Bsp. Nr. | $R^1$ | $R^2$ | n | $R^3$ | $-A-\overset{O}{\overset{\|\|}{C}}-X-R^4$ | Ar | physikalische Eigenschaften |
|---|---|---|---|---|---|---|---|
| 6 | $CH_3$ | $CF_3$ | 0 | H | $-CH_2-COOC_2H_5$ | 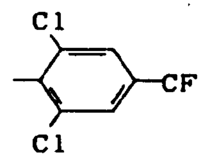 | $^1H$-NMR*): 7,77 |
| 7 | $CH_3$ | $CF_3$ | 0 | H | $-CH_2-COO-(CH_2)_2-CH_3$ | 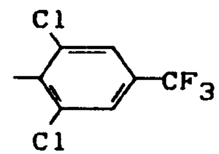 | $^1H$-NMR*): 7,78 |
| 8 | $CH_3$ | $CF_3$ | 0 | H | $-CH_2-COO-CH(CH_3)_2$ | 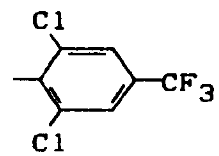 | $^1H$-NMR*): 7,78 |
| 9 | $CH_3$ | $CF_3$ | 0 | H | $-CH_2-COO-CH_2-$ 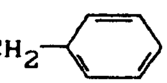 | 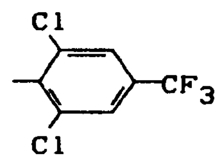 | $^1H$-NMR*): 7,72 |
| 10 | $CH_3$ | $CF_3$ | 0 | H | $-CH_2-CO-N(CH_3)_2$ | 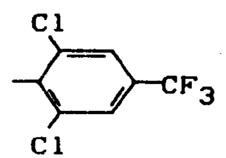 | Fp 109 °C |

**Fortsetzung:**

| Bsp. Nr. | $R^1$ | $R^2$ | n | $R^3$ | $-A-\overset{O}{\overset{\|}{C}}-X-R^4$ | Ar | physikalische Eigenschaften |
|---|---|---|---|---|---|---|---|
| 11 | H | $CF_3$ | 0 | $-\overset{O}{\overset{\|}{C}}-CH_3$ | $-COOC_2H_5$ | 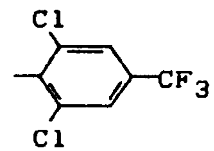 | Fp 70-73° C |
| 12 | H | $CF_3$ | 0 | H | -COOH | 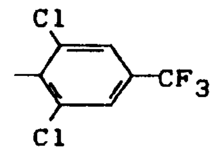 | Fp 97 °C (Zers.) |
| 13 | H | $CF_3$ | 0 | H | -COOH | 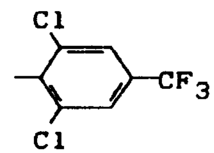 | Fp 101-103 °C |
| 14 | H | $CF_3$ | 1 | H | $-(CH_2)_3-COOH$ | 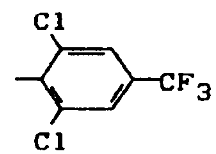 | $^1$H-NMR*): 7,90 |
| 15 | H | $CF_3$ | 2 | H | $-(CH_2)_3-COOH$ | 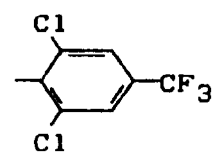 | Fp: 98-101° C |

**Fortsetzung:**

| Bsp. Nr. | $R^1$ | $R^2$ | n | $R^3$ | $-A-\overset{O}{\overset{\|}{C}}-X-R^4$ | Ar | physikalische Eigenschaften |
|---|---|---|---|---|---|---|---|
| 16 | H | $-CCl_2F$ | 1 | H | $-(CH_2)_3-COOH$ | 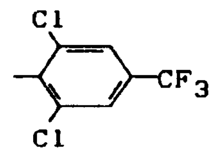 | $^1H$-NMR*) 7,85 |
| 17 | H | $-CCl_2F$ | 2 | H | $-(CH_2)_3-COOH$ | 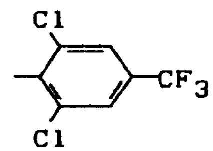 | Fp: 108-116° C |
| 18 | H | $CF_3$ | 0 | H | $-(CH_2)_3-COOC_2H_5$ | 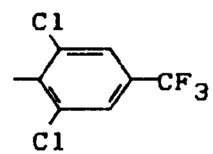 | $^1H$-NMR*) 7,67 |
| 19 | H | $-CCl_2F$ | 0 | H | $-(CH_2)_3-COOC_2H_5$ | 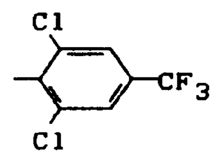 | $^1H$-NMR*) 7,72 |
| 20 | H | $-CCl_2F$ | 0 | $-\overset{O}{\overset{\|}{C}}-CH_3$ | $-CH_2-COOC_2H_5$ | 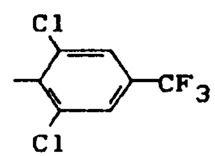 | Fp:104-107° C |

**Fortsetzung:**

| Bsp. Nr. | $R^1$ | $R^2$ | n | $R^3$ | $-A-\overset{\overset{O}{\|}}{C}-X-R^4$ | Ar | physikalische Eigenschaften |
|---|---|---|---|---|---|---|---|
| 21 | H | $-CCl_2F$ | 1 | H | $-(CH_2)_3-COOC_2H_5$ | 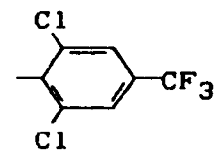 | $^1$H-NMR*) 7,91 |
| 22 | H | $-CCl_2F$ | 2 | H | $-(CH_2)_3-COOC_2H_5$ | 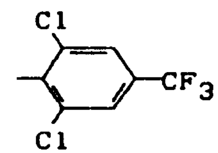 | $^1$H-NMR*) 7,89 |
| 23 | H | $-CCl_2F$ | 0 | H | $-CH_2-COOH$ | 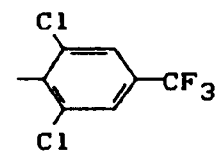 | Fp:156-159° C |
| 24 | H | $CF_3$ | 1 | H | $-(CH_2)_3-COOC_2H_5$ | 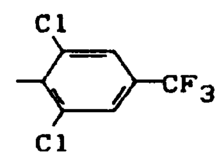 | $^1$H-NMR*) 7,95 |
| 25 | H | $CF_3$ | 2 | H | $-(CH_2)_3-COOC_2H_5$ | 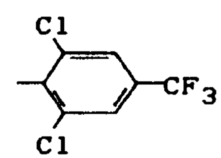 | $^1$H-NMR*) 7,87 |

Fortsetzung:

| Bsp. Nr. | $R^1$ | $R^2$ | n | $R^3$ | $-A-\overset{O}{\overset{\parallel}{C}}-X-R^4$ | Ar | physikalische Eigenschaften |
|---|---|---|---|---|---|---|---|
| 26 | H | $CF_3$ | 0 | H | $-CH_2-COOC_2H_5$ | 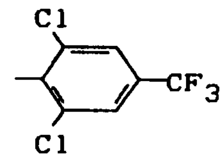 | Fp: 64-70° C |
| 27 | H | $-CCl_2F$ | 0 | H | $-CH_2-COOC_2H_5$ | 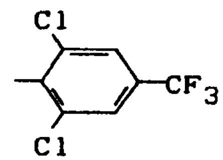 | |

*) Die $^1H$-NMR-Spektren wurden in Deuterochloroform ($CDCl_3$) mit Tetramethylsilan (TMS) als innerem Standard aufgenommen. Angegeben ist die chemische Verschiebung der Phenyl-Protonen im Ar-Substituenten als δ-Wert in ppm.

Anwendungsbeispiele:

In den folgenden Anwendungsbeispielen werden die nachstehend aufgeführten Verbindungen als Vergleichssubstanzen eingesetzt:

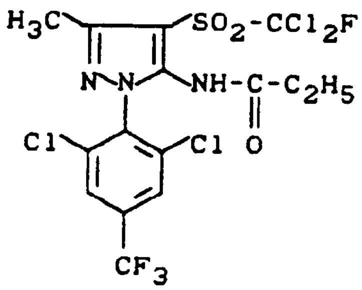

(A)

1-(2,6-Dichlor-4-trifluormethylphenyl)-3-methyl-4-dichlorfluormethylsulfonyl-5-propionamidopyrazol

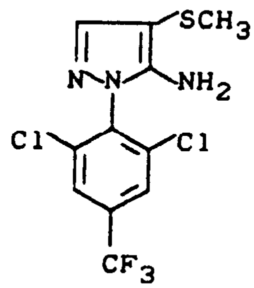

(B)

5-Amino-1-(2,6-dichlor-4-trifluormethylphenyl)-4-methylthio-pyrazol

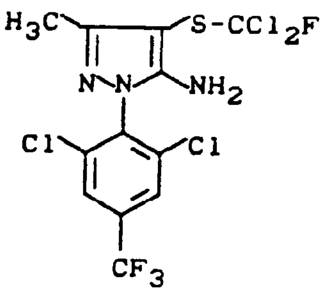

(C)

5-Amino-1-(2,6-dichlor-4-trifluormethylphenyl)-3-methyl-4-dichlormethylthio-pyrazol

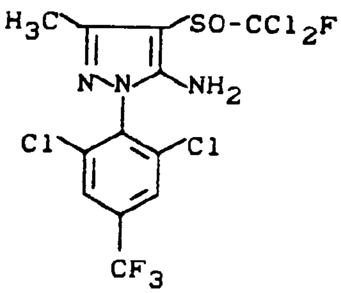

(D)

5-Amino-1-(2,6-dichlor-4-trifluormethylphenyl)-3-methyl-4-dichlorfluormethylsulfinyl-pyrazol

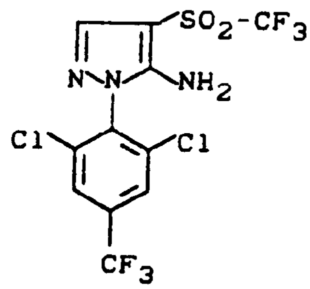

(E)

5-Amino-1-(2,6-dichlor-4-trifluormethylphenyl)-4-trifluormethylsulfonyl-pyrazol
(alle bekannt aus EP 201 852)

Beispiel A

Myzus-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 3, 13, 23 und 26.

Beispiel B

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 3, 26 und 27.

Beispiel C

Spodoptera-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter

noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 16, 17, 23, 26 und 27.

Beispiel D

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zübereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 2, 3, 4, 6, 7, 8, 9, 10, 14 und 17.

Beispiel E

Grenzkonzentrations-Test / Bodeninsekten

Testinsekt: Diabrotika balteata - Larven (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Die Wirkstoffzubereitung wird innig mit dem Boden vermischt. Dabei spielt die Konzentration des Wirkstoffes in der Zübereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei 20°C stehen.

Sofort nach dem Ansatz werden je Topf 6 vorgekeimte Maiskörner gelegt. Nach 2 Tagen werden in den behandelten Boden die entsprechendenm Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.

Bei diesem Test zeigen z.B. die folgenden Verbindungen der Herstellungsbeispiele überlegene Wirksamkeit gegenüber dem Stand der Technik: 2 und 3.

Beispiel F

Grenzkonzentrations-Test / Wurzelsystemische Wirkung

Testinsekt: Phaedon cochleariae - Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 1, 2, 3, 4, 6, 7, 8, 9 10, 12, 14 und 16.

Beispiel G

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 3 uns 12.

Beispiel H

Saatgutbehandlungs-Test/Bodeninsekten

| | |
|---|---|
| Testinsekt: | Phorbia antiqua-Maden im Boden |
| Testpflanze: | Allium cepa |
| Lösungsmittel: | 1 Gewichsteil Aceton |
| Trägermaterial: | Kaolin |

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Zwiebelsaatgut wird mit dieser Wirkstoffzubereitung mit den geforderten Aufwandmengen behandelt. Die Aussaat erfolgt in 0,5-l-Töpfen mit standardisierten Böden bei 20 °C Gewächshaustemperatur.

Nach Auflauf der Zwiebeln werden diese mit Zwiebelfliegeneiern künstlich infiziert.

Die Auswertung erfolgt nach 14 Tagen. Der Wirkungsgrad ist 100 %, wenn alle Zwiebelpflanzen stehenbleiben, 0 %, wenn alle Testpflanzen (wie in der unbehandelten Kontrolle) vernichtet wurden.

Bei diesem Test zeigen z. B. die folgenden Verbindungen der Herstellungsbeispiele überlegene Wirkung gegenüber dem Stand der Technik: 2, 3 und 12.

Beispiel I

Saatgutbehandlungs-Test/Wurzelsystemische Wirkung

Testinsekt: Phaedon cochleariae-Käfer
Testpflanze: Brassica oleracea
Lösungsmittel: 1 Gewichtsteil Aceton
Trägermaterial: Kaolin

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Kohl-Saatgut wird mit dieser Wirkstoffzubereitung mit den geforderten Aufwandmengen behandelt. Die Kohl-Aussaat erfolgt in 0,5-l-Töpfen mit standardisierten Böden bei 20 °C Raumtemperatur.

Der Wirkstoff kann so von den Pflanzenwurzeln aus dem Boden aufgenommen und in die Blätter transportiert werden.

Für den Nachweis des wurzelsystemischen Effektes werden nach 14 Tagen ausschließlich die Blätter mit den obengenannten Testtieren besetzt. Nach weiteren 3 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 2, 3 und 12.

Beispiel: K

Zeckentest (Boophilus microplus)/Hemmung der Eiablage

Lösungsmittel : 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.
In dieser Wirkstoffzubereitung werden adulte, vollgesogene Zeckenweibchen der Arten boophilus microplus (sensibel bzw. resistent) eine Minute lang getaucht. Nach dem Tauchen von je 10 weiblichen Exemplaren der verschiedenen Zeckenarten überführt man diese in Petrischalen, deren Boden mit einer entsprechend großen Filterscheibe belegt ist.

Nach 10 Tagen wird die Wirksamekit der Wirkstoffzubereitung bestimmt durch Ermittelung der Hemmung der Eiablage gegenüber unbehandelten Kontrollzecken. Die Wirkung drückt man in Prozent aus, wobei 100 % bedeutet, daß keine Eier mehr abgelegt wurden und 0 % besagt, daß die Zecken Eier in normaler Menge ablegen.

Bei diesem Test zeigen z.B. die folgenden Verbindungen der Herstellungsbeispiele überlegene Wirkung gegenüber dem Stand der Technik: 1 und 20.

**Patentansprüche**

1. Substituierte 1-Arylpyrazole der allgemeinen Formel (I)

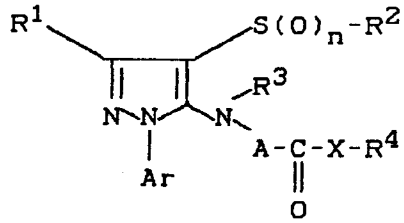

(I)

in welcher

$R^1$ für Wasserstoff oder für geradkettiges oder verzweigtes Alkyl mit 1 bis 6 Kohlenstoffatomen steht,

$R^2$ für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^3$ für Wasserstoff oder für geradkettiges oder verzweigtes Alkanoyl mit 1 bis 5 Kohlenstoffatomen steht,

$R^4$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und außerdem für den Fall, daß X für Sauerstoff oder Schwefel steht, auch für ein Äquivalent eines Alkali-, Erdalkali-, Kupfer-, Zink-, Mangan-, Zinn-, Eisen-Cobalt- oder Nickelkations oder für ein gegebenenfalls einfach oder mehrfach, gleich oder verschieden substituiertes Ammonium-, Phosphonium-oder Sulfoniumkation steht, wobei als Substituenten infrage kommen: geradkettiges oder verzweigtes Alkyl mit 1 bis 18 Kohlenstoffatomen, Phenyl oder Benzyl,

A für einen zweifach verknüpften, geradkettigen oder verzweigten Alkylenrest mit 1 bis 12 Kohlenstoffatomen steht,

X für Sauerstoff, Schwefel oder für einen Rest

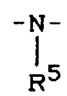

steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^6$ und,

n für eine Zahl 0, 1 oder 2 steht, wobei

$R^5$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht,

wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^6$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht.

**2.** Substituierte 1-Arylpyrazole der allgemeinen Formel (I) gemäß Anspruch 1, in welcher

$R^1$ für Wasserstoff, Methyl, Ethyl, n- oder i- Propyl, n-, i-, s- oder t-Butyl steht,

$R^2$ für Methyl, Ethyl, n- oder i-Propyl, n-, i-, s-oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder Brompropyl steht,

$R^3$ für Wasserstoff, Acetyl, Propionyl oder Butyryl steht,

$R^4$ für Wasserstoff, für gegebenenfalls ein- bis dreifach, gleich oder verschieden durch Fluor, Chlor, Hydroxy, Methoxy, Ethoxy, Methylthio oder Ethylthio substituiertes Methyl, Ethyl, n- oder i-Propyl, n-, i-, s- oder t-Butyl oder n- oder i-Pentyl, für gegebenenfalls ein- bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, und außerdem für den Fall, daß X für Sauerstoff oder Schwefel steht, auch für ein Äquivalent eines Natrium-, Kalium-, Magnesium-, Calcium-, Barium-, Kupfer-, Zink-, Mangan-, Zinn-, Eisen-, Cobalt- oder Nickelkations oder für ein gegebenenfalls ein-bis vierfach, gleich oder verschieden durch Methyl, Ethyl, n-oder i-Propyl, n-, i-, s-oder t-Butyl, Benzyl oder Phenyl substituiertes Ammonium-, Phosphonium- oder Sulfoniumkation steht,

A für einen zweifach verknüpften, geradkettigen oder verzweigten Alkylenrest mit 1 bis 6 Kohlenstoffatomen steht,

X für Sauerstoff, Schwefel oder für einen Rest

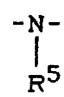

steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^6$ und

n für eine Zahl 0, 1 oder 2 steht, wobei

$R^5$ für Wasserstoff, für gegebenenfalls ein- bis dreifach, gleich oder verschieden durch Fluor, Chlor, Hydroxy, Methoxy, Ethoxy, Methylthio oder Ethylthio substituiertes Methyl, Ethyl, n- oder i-Propyl, n-, i-, s- oder t-Butyl oder n- oder i-Pentyl, für gegebenenfalls ein- bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht,

$R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,

p für eine Zahl 0, 1 oder 2 steht.

**3.** Substituierte 1-Arylpyrazole der allgemeinen Formel (I) gemäß Anspruch 1, in welcher

$R^1$ für Wasserstoff oder Methyl steht,

$R^2$ für Trifluormethyl, Dichlorfluormethyl oder Chlordifluormethyl steht,

$R^3$ für Wasserstoff, Acetyl oder Propionyl steht,

$R^4$ für Wasserstoff, Methyl, Ethyl, n- oder i- Propyl, n-, i-, s- oder t-Butyl oder Benzyl steht,

A für einen Rest der Formel $-CH_2-$; $-CH_2-CH_2-$; $-CH_2-CH_2-CH_2-$ oder

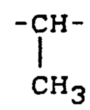

steht,

X für Sauerstoff oder für einen Rest

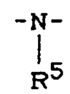

steht,

Ar für gegebenenfalls ein- bis fünffach, gleich oder verschieden durch Fluor, Chlor, Brom, Trifluormethyl oder Trifluormethoxy substituiertes Phenyl steht und

n für eine Zahl 0, 1 oder 2 steht, wobei

$R^5$ für Methyl, Ethyl, n- oder i-Propyl, n-, i-, s- oder t-Butyl oder für einen Rest $-SO_2-R^6$ steht und

$R^6$ für Methyl, Trifluormethyl, Phenyl oder p-Tolyl steht.

**4.** Verfahren zur Herstellung von 1-Arylpyrazolen der allgemeinen Formel (I)

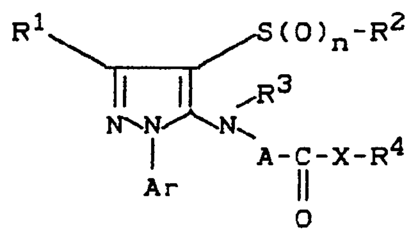

(I)

in welcher

$R^1$, $R^2$, $R^3$, $R^4$, A, X, Ar und n die in Anspruch 1 angegebene Bedeutung haben,

(a) dadurch gekennzeichnet, daß man zum Erhalt von substituierten 1-Arylpyrazolen der Formel (I),

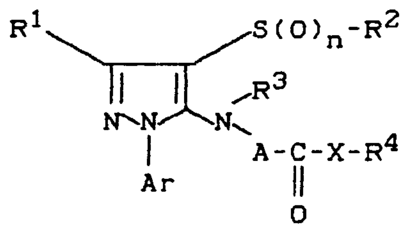

(I)

in welcher

$R^1$, $R^2$, $R^3$, $R^4$, A, X, Ar und n die oben angegebene Bedeutung haben,

1-Arylpyrazole der Formel (II),

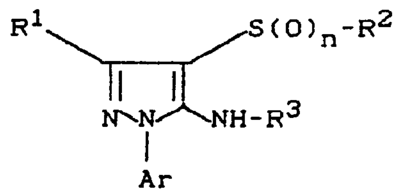

(II)

in welcher

$R^1$, $R^2$, $R^3$, Ar und n die oben angegebene Bedeutung haben,

mit Alkylierungsmitteln der Formel (III),

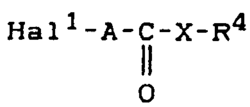

(III)

in welcher

$Hal^1$ für Chlor, Brom oder Jod steht und

$R^4$, A und X die oben angegebene Bedeutung haben,

gegebenenfalls in Gegenwart eines Verdünnungsmittels und gegebenenfalls in Gegenwart eines Reaktionshilfsmittels umsetzt;

(b) oder, daß man zum Erhalt von substituierten 1-Arylpyrazolen der Formel (Ib),

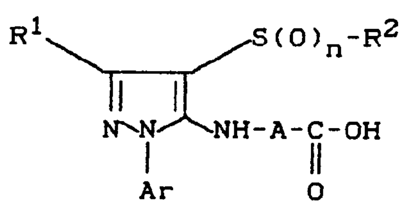

(Ib)

in welcher

$R^1$, $R^2$, A, Ar und n die oben angegebene Bedeutung haben,

1-Arylpyrazole der Formel (Ia),

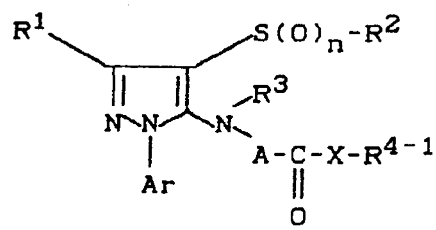

(Ia)

in welcher

$R^{4-1}$ für für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$, $R^2$, $R^3$, A, X, Ar und n die oben angegebene Bedeutung haben,

mit Säuren oder Basen als Katalysator in Gegenwart von Wasser sowie gegebenenfalls in Gegenwart eines Verdünnungsmittels verseift;

(c) man erhält substituierte 1-Arylpyrazole der Formel (Ic),

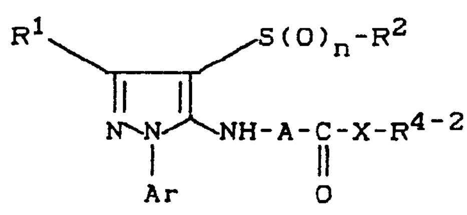

(Ic)

in welcher

$R^{4-2}$ für Wasserstoff, für jeweils geradkettiges oder verzweigtes Alkyl oder Hydroxyalkyl mit jeweils 1 bis 12 Kohlenstoffatomen, für jeweils geradkettiges oder verzweigtes Alkoxyalkyl oder Alkylthioalkyl mit jeweils 2 bis 12 Kohlenstoffatomen, für jeweils geradkettiges oder verzweigtes Alkenyl oder Alkinyl mit jeweils 2 bis 12 Kohlenstoffatomen, für geradkettiges oder verzweigtes Halogenalkyl mit 1 bis 8 Kohlenstoffatomen und 1 bis 17 gleichen oder verschiedenen Halogenatomen für geradkettiges oder verzweigtes Halogenalkenyl mit 2 bis 8 Kohlenstoffatomen und 1 bis 15 gleichen oder verschiedenen Halogenatomen, für gegebenenfalls einfach

oder mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen oder für jeweils im Phenylteil gegebenenfalls einfach oder mehrfach, gleich oder verschieden substituiertes Phenyl, Benzyl oder Phenylethyl steht, wobei als Phenylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und

$R^1$, $R^2$, A, X, Ar und n die oben angegebene Bedeutung haben,

substituierte 1-Arylpyrazole der Formel (Ib),

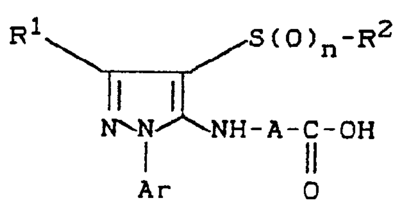

(Ib)

in welcher

$R^1$, $R^2$, A und n die oben angegebene Bedeutung haben,

mit Alkoholen, Thiolen oder Aminoverbindungen der Formel (IV),

$$R^{4-2}\text{-X-H} \qquad \text{(IV)}$$

in welcher

$R^{4-2}$ und X die oben angegebene Bedeutung haben,

in Gegenwart eines Acylierungskatalysators sowie gegebenenfalls in Gegenwart eines Verdünnungsmittels und gegebenenfalls in Gegenwart eines Reaktionshilfsmittels umsetzt;

(d) oder, daß man zum Erhalt von substituierten 1-Arylpyrazolen der Formel (Id),

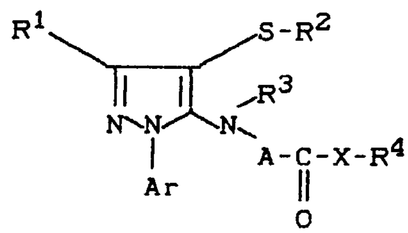

(Id)

in welcher

$R^1$, $R^2$, $R^3$, $R^4$, A, X und Ar die oben angegebene Bedeutung haben,

in 4-Stellung unsubstituierte 1-Arylpyrazole der Formel (V),

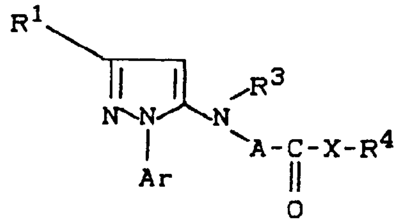

(V)

in welcher

$R^1$, $R^3$, $R^4$, A, X und Ar die oben angegebene Bedeutung haben,

mit Sulfenylhalogeniden der Formel (VI),

$$R^2\text{-S-Hal}^2 \quad \text{(VI)}$$

in welcher

$Hal^2$ für Chlor oder Brom steht und

$R^2$ die oben angegebene Bedeutung hat,

gegebenenfalls in Gegenwart eines Verdünnungsmittels und gegebenenfalls in Gegenwart eines Reaktionshilfsmittels umsetzt;

(e) oder, daß man zum Erhalt von substituierten 1-Arylpyrazolen der Formel (Ie),

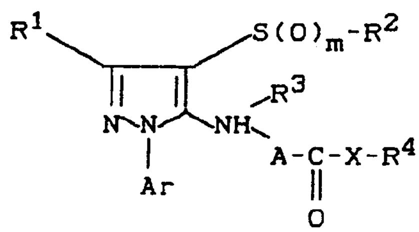

(Ie)

in welcher

$R^1$, $R^2$, $R^3$, $R^4$, A, X und Ar die oben angegebene Bedeutung haben und

m für eine Zahl 1 oder 2 steht,

substituierte 1-Arylpyrazole der Formel (Id),

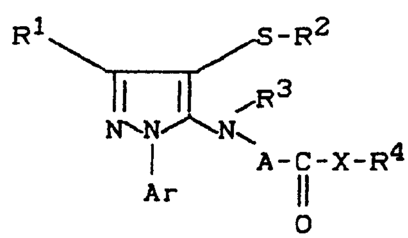

(Id)

in welcher

$R^1$, $R^2$, $R^3$, $R^4$, A, X und Ar die oben angegebene Bedeutung haben,

mit Oxidationsmitteln gegebenenfalls in Gegenwart eines Verdünnungsmittels und gegebenenfalls in Gegenwart eines Katalysators umsetzt;

(f) oder, daß man zum Erhalt von substituierten 1-Arylpyrazolen der Formel (If),

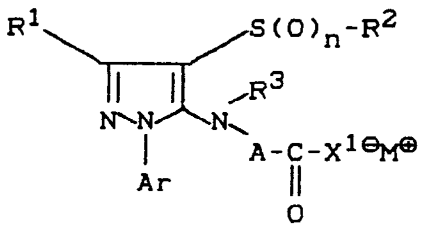

(If)

in welcher

$X^1$ für Sauerstoff oder Schwefel steht,

$M^{\oplus}$ für ein salzartig gebundenes Kation steht und

$R^1$, $R^2$, $R^3$, A, Ar und n die oben angegebene Bedeutung haben,

substituierte 1-Arylpyrazole der Formel (Ig),

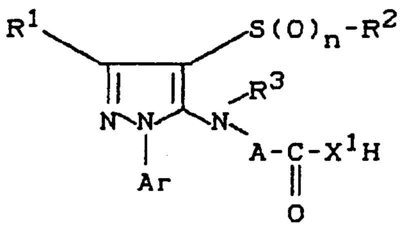

(Ig)

in welcher

$R^1$, $R^2$, $R^3$, A, $X^1$, Ar und n die oben angegebene Bedeutung haben,

mit anorganischen oder organischen Basen gegebenenfalls in Gegenwart eines Verdünnungsmittels umsetzt.

5. Schädlingsbekämpfungsmittel, gekennzeichnet durch einen Gehalt an mindestens einem 1-Arylpyrazol der Formel (I) gemäß Anspruch 1.

6. Insektizide Mittel, gekennzeichnet durch einen Gehalt an mindestens einem 1-Arylpyrazol der Formel (I) gemäß Anspruch 1.

7. Verfahren zur Bekämpfung von Insekten, dadurch gekennzeichnet, daß man 1-Arylpyrazole der Formel (I) gemäß Anspruch 1 auf Insekten und/oder deren Lebensraum einwirken läßt, ausgenommen die Bekämpfung von Ektoparasiten.

8. Verwendung von 1-Arylpyrazolen der Formel (I) gemäß Anspruch 1 zur Bekämpfung von Insekten, ausgenommen die Bekämpfung von Ektoparasiten.

9. Verfahren zur Herstellung von insektiziden Mitteln, dadurch gekennzeichnet, daß man 1-Arylpyrazole der Formel (I) gemäß Anspruch 1 mit Streckmitteln und/oder oberflächenaktiven Mitteln vermischt.

10. Verwendung von 1-Arylpyrazolen der Formel (I) gemäß Anspruch 1 zur Herstellung von Mitteln zur Bekämpfung von Ektoparasiten.

## Claims

1. Substituted 1-arylpyrazoles of the general formula (I)

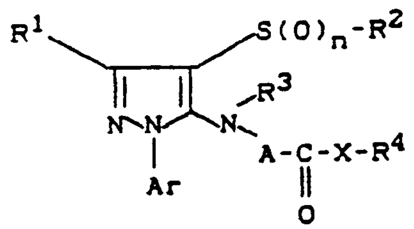

(I)

in which

- $R^1$ represents hydrogen or straight-chain or branched alkyl having 1 to 6 carbon atoms,
- $R^2$ represents straight-chain or branched alkyl having 1 to 6 carbon atoms or straight-chain or branched halogenoalkyl having 1 to 4 carbon atoms and 1 to 9 identical or different halogen atoms,
- $R^3$ represents hydrogen or straight-chain or branched alkanoyl having 1 to 5 carbon atoms,
- $R^4$ represents hydrogen, in each case straight-chain or branched alkyl or hydroxyalkyl, each of which has 1 to 12 carbon atoms, in each case straight-chain or branched alkoxyalkyl or alkylthioalkyl, each of which has 2 to 12 carbon atoms, in each case straight-chain or branched alkenyl or alkinyl, each of which has 2 to 12 carbon atoms, straight-chain or branched halogenoalkyl having 1 to 8 carbon atoms and 1 to 17 identical or different halogen atoms, straight-chain or branched halogenoalkenyl having 2 to 8 carbon atoms and 1 to 15 identical or different halogen atoms, cycloalkyl which has 3 to 7 carbon atoms and which is optionally monosubstituted or polysubstituted by identical or different straight-chain or branched alkyl substituents having 1 to 4 carbon atoms, or represents phenyl, benzyl or phenylethyl, each of which is optionally monosubstituted or polysubstituted in the phenyl moiety by identical or different substituents, suitable phenyl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties and phenyl which is optionally monosubstituted or polysubstituted by identical or different substituents from the series comprising halogen and/or straight-chain or branched alkyl having 1 to 4 carbon atoms; and additionally in the case when X represents oxygen or sulphur, $R^4$ also represents an equivalent of an alkali metal cation, alkaline earth metal cation, copper cation, zinc cation, manganese cation, tin cation, iron cation, cobalt cation or nickel cation, or an ammonium, phosphonium or sulphonium cation which is optionally monosubstituted or polysubstituted by identical or different substituents, suitable substituents being: straight-chain or branched alkyl having 1 to 18 carbon atoms, phenyl or benzyl,
- A represents a divalent straight-chain or branched alkylene radical having 1 to 12 carbon atoms,
- X represents oxygen, sulphur or a radical

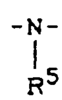

- Ar represents phenyl, 2-pyridyl, 3-pyridyl or 4-pyridyl, each of which is optionally monosubstituted or polysubstituted by identical or different substituents, suitable substituents in each case being: cyano, nitro, halogen, in each case straight-chain or branched alkyl, alkoxy or alkoxycarbonyl, each of which has 1 to 4 carbon atoms, in each case straight-chain or branched halogenoalkyl or halogenoalkoxy, each of which has 1 to 4 carbon atoms and 1 to 9 identical or different halogen atoms or a radical $-S(O)_p-R^6$ and
- n represents a number 0, 1 or 2, where
  - $R^5$ represents hydrogen, in each case straight-chain or branched alkyl or hydroxyalkyl, each of which has 1 to 12 carbon atoms, in each case straight-chain or branched alkoxyalkyl or alkylthioalkyl, each of which has 2 to 12 carbon atoms, in each case straight-chain or branched alkenyl or alkinyl, each of which has 2 to 12 carbon atoms, straight-chain or

branched halogenoalkyl having 1 to 8 carbon atoms and 1 to 17 identical or different halogen atoms, straight-chain or branched halogenoalkenyl having 2 to 8 carbon atoms and 1 to 15 identical or different halogen atoms, cycloalkyl which has 3 to 7 carbon atoms and which is optionally monosubstituted or polysubstituted by identical or different straight-chain or branched alkyl substituents having 1 to 4 carbon atoms, or phenyl, benzyl or phenylethyl, each of which is optionally monosubstituted or polysubstituted in the phenyl moiety by identical or different substituents, suitable phenyl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties as well as phenyl which is optionally monosubstituted or polysubstituted by identical or different substituents from the series comprising halogen and/or straight-chain or branched alkyl having 1 to 4 carbon atoms;

$R^6$ represents amino as well as in each case straight-chain or branched alkyl, alkylamino, dialkylamino or halogenoalkyl, each of which has 1 to 4 carbon atoms in the individual alkyl moieties and, in the case of halogenoalkyl, 1 to 9 identical or different halogen atoms, and

p represents a number 0, 1 or 2.

**2.** Substituted 1-arylpyrazoles of the general formula (I) according to Claim 1, in which

$R^1$ represents hydrogen, methyl, ethyl, n- or i-propyl or n-, i-, s- or t-butyl,

$R^2$ represents methyl, ethyl, n- or i-propyl, n-, i-, s- or t-butyl, chloromethyl, difluoromethyl,difluorochloromethyl,fluorodichloromethyl, trifluoromethyl, pentafluoroethyl, pentachloroethyl, fluorotetrachloroethyl, difluorotrichloroethyl, trifluorodichloroethyl, tetrafluorochloroethyl, heptafluoropropyl, chloroethyl, bromoethyl, chloropropyl or bromopropyl,

$R^3$ represents hydrogen, acetyl, propionyl or butyryl,

$R^4$ represents hydrogen, methyl, ethyl, n- or i-propyl, n-, i-, s- or t-butyl or n- or i-pentyl, each of which is optionally monosubstituted to trisubstituted by identical or different substituents from the series comprising fluorine, chlorine, hydroxyl, methoxy, ethoxy, methylthio or ethylthio, or represents allyl, propenyl or butenyl, each of which is optionally monosubstituted to trisubstituted by identical or different substituents from the series comprising fluorine or chlorine, or represents propargyl, propinyl, butinyl or pentinyl, or represents cyclopropyl, cyclopentyl, cyclohexyl, or represents benzyl or phenyl each of which is optionally monosubstituted to pentasubstituted by identical or different substituents from the series comprising fluorine, chlorine, bromine, cyano, nitro, methyl, ethyl, methoxy, ethoxy, methylthio or trifluoromethyl, and furthermore in the event that X represents oxygen or sulphur, $R^4$ also represents an equivalent of a sodium cation, potassium cation, magnesium cation, calcium cation, barium cation, copper cation, zinc cation, manganese cation, tin cation, iron cation, cobalt cation or nickel cation or an ammonium cation, phosphonium cation or sulphonium cation, each of which is optionally monosubstituted to tetrasubstituted by identical or different substituents from the series comprising methyl, ethyl, n- or i-propyl, n-, i-, s- or t-butyl, benzyl or phenyl,

A represents a divalent straight-chain or branched alkylene radical having 1 to 6 carbon atoms,

X represent oxygen, sulphur or a radical

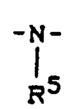

Ar represents phenyl which is optionally monosubstituted to pentasubstituted by identical or different substituents or 2-pyridyl which is optionally monosubstituted to tetrasubstituted by identical or different substituents, suitable substituents in each case being: cyano, nitro, fluorine, chlorine, bromine, iodine, methyl, ethyl, n- and i-propyl, n-, i-, s- and t-butyl, methoxy, ethoxy, methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or a radical $-S(O)_p-R^6$ and

n represents a number 0, 1 or 2, in which

$R^5$ represents hydrogen, methyl, ethyl, n- or i-propyl, n-, i-, s- or t-butyl or n- or i-pentyl, each of which is optionally monosubstituted to trisubstituted by identical or different substituents from the series comprising fluorine, chlorine, hydroxyl, methoxy, ethoxy, methylthio or ethylthio, or represents allyl, propenyl or butenyl, each of which is optionally monosubstituted to trisubstituted by identical or different substituents from the series comprising fluorine or chlorine, or represents propargyl, propinyl, butinyl or pentinyl, or represents cyclopropyl, cyclopentyl or cyclohexyl, or represents benzyl or phenyl, each of which is optionally monosubstituted to pentasubstituted by identical or different substituents from the series comprising fluorine, chlorine, bromine, cyano, nitro, methyl, ethyl, methoxy, ethoxy, methylthio or trifluoromethyl,

$R^6$ represents amino, methylamino, ethylamino, dimethylamino, diethylamino, fluorodichloromethyl, difluoromethyl, tetrafluoroethyl, trifluorochloroethyl, trichloromethyl, trichloroethyl, trifluoromethyl, methyl or ethyl, and

p represents a number 0, 1 or 2.

**3.** Substituted 1-arylpyrazoles of the general formula (I) according to Claim 1, in which

$R^1$ represents hydrogen or methyl,

$R^2$ represents trifluoromethyl, dichlorofluoromethyl, or chlorodifluoromethyl,

$R^3$ represents hydrogen, acetyl or propionyl,

$R^4$ represents hydrogen, methyl, ethyl, n- or i-propyl, n-, i-, s- or t-butyl or benzyl,

A represents a radical of the formula $-CH_2-$; $-CH_2-CH_2-$; $-CH_2-CH_2-CH_2-$ or

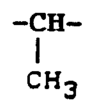

X represents oxygen or a radical

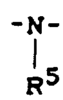

Ar represents phenyl which is optionally monosubstituted to pentasubstituted by identical or different substituents from the series comprising fluorine, chlorine, bromine, trifluoromethyl or trifluoromethoxy and

n represents a number 0, 1 or 2, in which

$R^5$ represents methyl, ethyl, n- or i-propyl, n-, i-, s- or t-butyl or a radical $-SO_2-R^6$ and

$R^6$ represents methyl, trifluoromethyl, phenyl or p-tolyl.

**4.** Process for the preparation of 1-arylpyrazoles of the general formula (I)

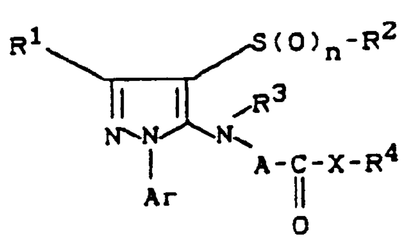

(I)

in which

$R^1$, $R^2$, $R^3$, $R^4$, A, X Ar and n have the meaning given in Claim 1,

(a) characterised in that, to obtain substituted 1-arylpyrazoles of the formula (I),

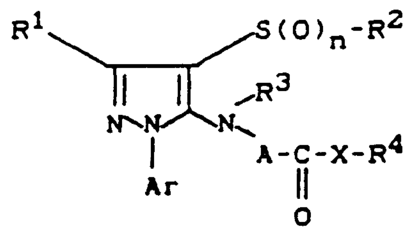

(I)

in which $R^1$, $R^2$, $R^3$, $R^4$, A, X, Ar and n have the abovementioned meaning, 1-arylpyrazoles of the formula (II)

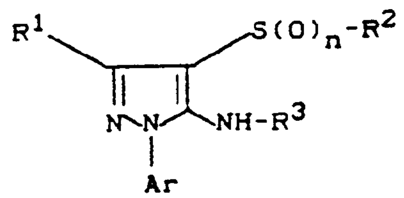

(II)

in which $R^1$, $R^2$, $R^3$, Ar and n have the abovementioned meaning, are reacted with alkylating agents of the formula (III)

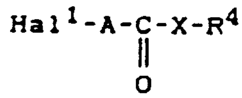

(III)

in which $Hal^1$ represents chlorine, bromine or iodine and $R^4$, A and X have the abovementioned meaning if appropriate in the presence of a diluent and if appropriate in the presence of a reaction auxiliary;
(b) or in that, to obtain substituted 1-arylpyrazoles of the formula (Ib),

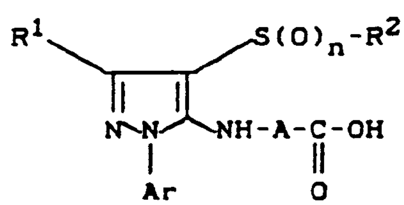

(Ib)

in which $R^1$, $R^2$, A, Ar and n have the abovementioned meaning, 1-arylpyrazoles of the formula (Ia)

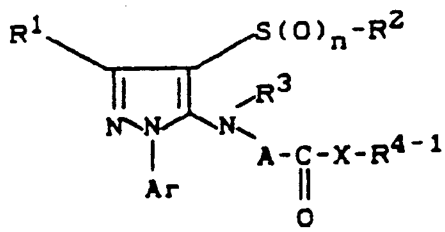

(Ia)

in which

$R^{4-1}$ represents in each case straight-chain or branched alkyl or hydroxyalkyl, each of which has 1 to 12 carbon atoms, in each case straight-chain or branched alkoxyalkyl or alkylthioalkyl, each of which has 2 to 12 carbon atoms, in each case straight-chain or branched alkenyl or alkinyl, each of which has 2 to 12 carbon atoms, in each case straight-chain or branched halogenoalkyl having 1 to 8 carbon atoms and 1 to 17 identical or different halogen atoms, or represents straight-chain or branched halogenoalkenyl having 2 to 8 carbon atoms and 1 to 15 identical or different halogen atoms, or represents cycloalkyl which has 3 to 7 carbon atoms and which is optionally monosubstituted or polysubstituted by identical or different straight-chain or branched alkyl substituents having 1 to 4 carbon atoms, or represents phenyl, benzyl or phenylethyl, each of which is optionally monosubstituted or polysubstituted in the phenyl moiety by identical or different substituents, suitable phenyl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, as well as phenyl which is optionally monosubstituted or polysubstituted by identical or different substituents from the series comprising halogen and/or straight-chain or branched alkyl having 1 to 4 carbon atoms; and

$R^1$, $R^2$, $R^3$, A, X, Ar and n have the abovementioned meaning,

are hydrolysed with acids or bases as catalysts in the presence of water and, if appropriate, in the presence of a diluent;

(c) substituted 1-arylpyrazoles of the formula (Ic)

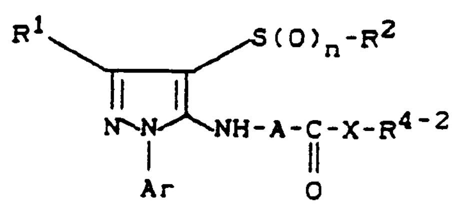

(Ic)

in which

$R^{4-2}$ represents hydrogen, in each case straight-chain or branched alkyl or hydroxyalkyl, each of which has 1 to 12 carbon atoms, in each case straight-chain or branched alkoxyalkyl or alkylthioalkyl, each of which has 2 to 12 carbon atoms, in each case straight-chain or branched alkenyl or alkinyl, each of which has 2 to 12 carbon atoms, in each case straight-chain or branched halogenoalkyl having 1 to 8 carbon atoms and 1 to 17 identical or different halogen atoms, or represents straight-chain or branched halogenoalkyl having 2 to 8 carbon atoms and 1 to 15 identical or different halogen atoms, or represents cycloalkyl which has 3 to 7 carbon atoms and which is optionally monosubstituted or polysubstituted by identical or different straight-chain or branched alkyl substituents having 1 to 4 carbon atoms, or represents phenyl, benzyl or phenylethyl, each of which is optionally monosubstituted or polysubstituted in the phenyl moiety by identical or different substituents, suitable phenyl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, as well as phenyl which is optionally monosubstituted or polysubstituted by identical or different substituents from the series comprising halogen and/or straight-chain or branched alkyl having 1 to 4 carbon atoms; and

$R^1$, $R^2$, $R^3$, A, X, Ar and n have the abovementioned meaning, are obtained by reacting substituted 1-arylpyrazoles of the formula (Ib)

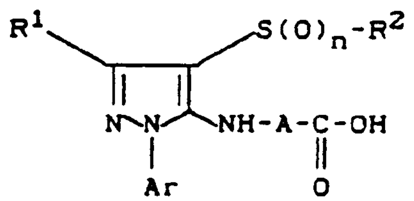

(Ib)

in which

$R^1$, $R^2$, A and n have the abovementioned meaning,

with alcohols, thiols or amino compounds of the formula (IV)

$$R^{4-2}\text{-X-H} \qquad (IV)$$

in which

$R^{4-2}$ and X have the abovementioned meaning,

in the presence of an acylating catalyst and, if appropriate, in the presence of a diluent and, if appropriate, in the presence of a reaction auxiliary;

(d) or in that, to obtain substituted 1-arylpyrazoles of the formula (Id),

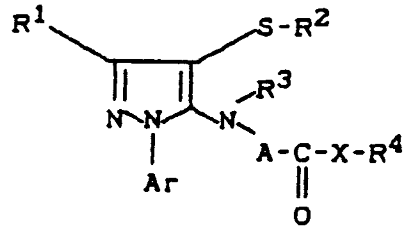

(Id)

in which

$R^1$, $R^2$, $R^3$, $R^4$, A, X and Ar have the abovementioned meaning,

1-arylpyrazoles of the formula (V), which are unsubstituted in the 4-position,

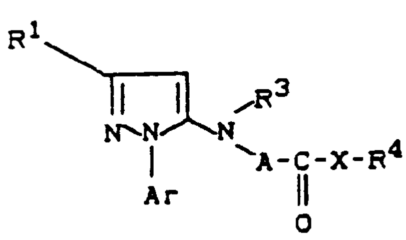

(V)

and in which

$R^1$, $R^3$, $R^4$, A, X and Ar have the abovementioned meaning,

are reacted with sulphenyl halides of the formula (VI)

$R^2$-S -$Hal^2$ (VI)

in which
$Hal^2$ represents chlorine or bromine and
$R^2$ has the abovementioned meaning,
if appropriate in the presence of a diluent and, if appropriate, in the presence of a reaction auxiliary;
(e) or in that, to obtain substituted 1-arylpyrazoles of the formula (Ie),

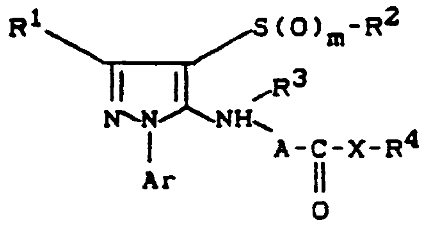

(Ie)

in which
$R^1$, $R^2$, $R^3$, $R^4$, A, X and Ar have the abovementioned meaning and
m represents a number 1 or 2,
substituted 1-arylpyrazoles of the formula ( Id),

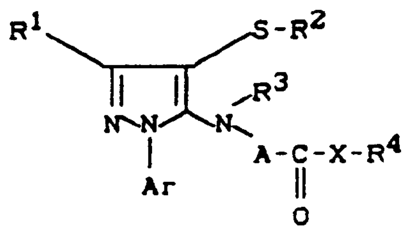

(Id)

in which
$R^1$, $R^2$, $R^3$, $R^4$, A, X and Ar have the abovementioned meaning,
are reacted with oxidants, if appropriate in the presence of a diluent and, if appropriate, in the presence of a catalyst;
(f) or in that, to obtain substituted 1-arylpyrazoles of the formula (If),

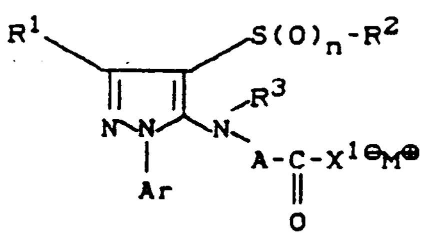

(If)

in which
$X^1$ represents oxygen or sulphur,
$M^{\oplus}$ represents a cation bonded in a salt-like fashion, and
$R^1$, $R^2$, $R^3$, A, Ar and n have the abovementioned meaning,
substituted 1-arylpyrazoles of the formula (Ig),

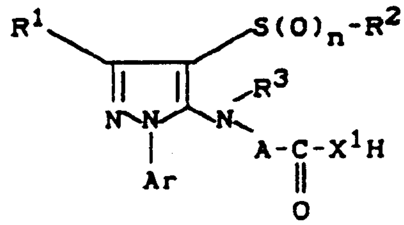

(Ig)

in which
$R^1$, $R^2$, $R^3$, A, $X^1$, Ar and n have the abovementioned meaning
are reacted with inorganic or organic bases, if appropriate in the presence of a diluent.

5. Pesticides, characterised in that they contain at least one 1-arylpyrazole of the formula (I) according to Claim 1.

6. Insecticidal agents, characterised in that they contain at least one 1-arylpyrazole of the formula (I) according to Claim 1.

7. Method of combating insects, characterised in that 1-arylpyrazoles of the formula (I) according to Claim 1 are allowed to act on insects and/or their environment, combating ectoparasites being excepted.

8. Use of 1-arylpyrazoles of the formula (I) according to Claim 1 for combating insects, combating ectoparasites being excepted.

9. Process for the preparation of insecticidal agents, characterised in that 1-arylpyrazoles of the formula (I) according to Claim 1 are mixed with extenders and/or surface-active agents.

10. Use of 1-arylpyrazoles of the formula (I) according to Claim 1 for the preparation of agents for combating ectoparasites.

## Revendications

1. 1-arylpyrazols substitués de formule générale (I)

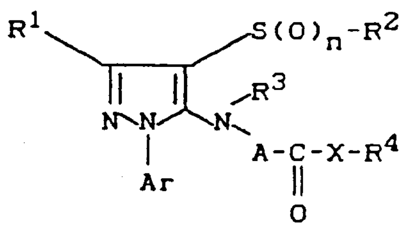

(I)

dans laquelle

$R^1$ représente l'hydrogène ou un groupe alkyle à chaîne droite ou ramifiée ayant 1 à 6 atomes de carbone,

$R^2$ est un groupe alkyle à chaîne droite ou ramifiée ayant 1 à 6 atomes de carbone ou un groupe halogénalkyle à chaîne droite ou ramifiée ayant 1 à 4 atomes de carbone et 1 à 9 atomes d'halogènes identiques ou différents,

$R^3$ est l'hydrogène ou un groupe alcanoyle à chaîne droite ou ramifiée ayant 1 à 5 atomes de carbone,

$R^4$ représente l'hydrogène, un groupe alkyle ou un groupe hydroxyalkyle ayant chacun 1 à 12 atomes de carbone à chaîne droite ou à chaîne ramifiée, un groupe alkoxyalkyle ou un groupe alkylthioalkyle ayant chacun 2 à 12 atomes de carbone à chaîne droite ou ramifiée, un groupe alcényle ou un groupe alcynyle ayant chacun 2 à 12 atomes de carbone à chaîne droite ou ramifiée, un groupe halogènalkyle à chaîne droite ou ramifiée ayant 1 à 8 atomes de carbone et 1 à 17 atomes

d'halogènes identiques ou différents, un groupe halogénalcényle à chaîne droite ou ramifiée ayant 2 à 8 atomes de carbone et 1 à 15 atomes d'halogènes identiques ou différents, un groupe cycloalkyle de 3 à 7 atomes de carbone portant éventuellement un ou plusieurs substituants alkyle à chaîne droite ou ramifiée identiques ou différents ayant 1 à 4 atomes de carbone, ou un groupe phényle, benzyle ou phényléthyle portant chacun le cas échéant dans la partie phényle un ou plusieurs substituants identiques ou différents, en considérant dans chaque cas comme substituants du groupe phényle : un halogène, un radical cyano, nitro, alkyle, alkoxy ou alkylthio ayant chacun 1 à 4 atomes de carbone à chaîne droite ou ramifiée, un groupe halogénalkyle, halogénalkoxy ou halogénalkylthio étant chacun à chaîne droite ou ramifiée avec chacun 1 à 4 atomes de carbone et 1 à 9 atomes d'halogènes identiques ou différents, un groupe alkoxycarbonyle ou alkoximinoalkyle étant chacun à chaîne droite ou ramifiée avec chacun 1 à 4 atomes de carbone dans les parties alkyle individuelles ainsi qu'un groupe phényle portant éventuellement un ou plusieurs substituants, identiques ou différents, halogéno et/ou alkyle à chaîne droite ou ramifiée ayant 1 à 4 atomes de carbone ;

et en outre, au cas où X représente l'oxygène ou le soufre, un équivalent de cation de métal de alcalin, de métal alcalino-terreux, de cuivre, de zinc, de manganèse, d'étain, de fer, de cobalt ou de nickel ou un ion ammonium, phosphonium ou sulfonium portant chacun le cas échéant un ou plusieurs substituants identiques ou différents, en considérant comme substituants : un groupe alkyle à chaîne droite ou ramifiée ayant 1 à 18 atomes de carbone, phényle ou benzyle,

A est un reste alkylène secondaire à chaîne droite ou ramifiée ayant 1 à 12 atomes de carbone,

X représente l'oxygène, le soufre ou un reste

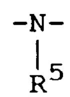

Ar représente un groupe phényle, 2-pyridyle, 3-pyridyle ou 4-pyridyle portant chacun le cas échéant un ou plusieurs substituants identiques ou différents, en considérant dans chaque cas comme substituants : un radical cyano, nitro, halogéno, un groupe alkyle, alkoxy ou alkoxycarbonyle à chaîne droite ou ramifiée avec chacun 1 à 4 atomes de carbone, un groupe halogénalkyle ou halogénalkoxy à chaîne droite ou ramifiée ayant chacun 1 à 4 atomes de carbone et 1 à 9 atomes d'halogènes identiques ou différents ou un reste $-S(O)_p-R^6$,

n représente le nombre 0, 1 ou 2,

$R^5$ est l'hydrogène, un groupe alkyle ou un groupe hydroxyalkyle chacun à chaîne droite ou ramifiée avec chacun 1 à 12 atomes de carbone, un groupe alkoxyalkyle ou un groupe alkylthioalkyle à chaîne droite ou ramifiée ayant chacun 2 à 12 atomes de carbone, un groupe alcényle ou un groupe alcynyle à chaîne droite ou ramifiée ayant chacun 2 à 12 atomes de carbone, un groupe halogénalkyle à chaîne droite ou ramifiée ayant 1 à 8 atomes de carbone et 1 à 17 atomes d'halogènes identiques ou différents, un groupe halogénalcényle à chaîne droite ou ramifiée ayant chacun 2 à 8 atomes de carbone et 1 à 15 atomes d'halogènes identiques ou différents, un groupe cycloalkyle de 3 à 7 atomes de carbone portant le cas échéant un ou plusieurs substituants, identiques ou différents, alkyle à chaîne droite ou ramifiée ayant 1 à 4 atomes de carbone, ou un groupe phényle, benzyle ou phényléthyle portant chacun dans la partie phényle le cas échéant un ou plusieurs substituants identiques ou différents, en considérant comme substituants du groupe phényle : un halogène, un radical cyano, nitro, un radical alkyle, alkoxy ou alkylthio à chaîne droite ou ramifiée ayant chacun 1 à 4 atomes de carbone, un radical halogénalkyle, halogénalkoxy ou halogénalkylthio à chaîne droite ou ramifiée ayant chacun 1 à 4 atomes de carbone et 1 à 9 atomes d'halogènes identiques ou différents, un groupe alkoxycarbonyle ou un groupe alkoximinoalkyle à chaîne droite ou ramifié ayant chacun 1 à 4 atomes de carbone dans les parties alkyle individuelles ainsi qu'un groupe phényle portant le cas échéant un ou plusieurs substituants halogéno et/ou alkyle en $C_1$ à $C_4$ à chaîne droite ou ramifiée, identiques ou différents ;

$R^6$ représente un groupe amino, ainsi qu'un groupe alkyle, alkylamino, dialkylamino ou halogénalkyle à chaîne droite ou ramifiée ayant chacun 1 à 4 atomes de carbone dans les parties alkyle individuelles et, dans le cas du groupe halogénalkyle, avec 1 à 9 atomes d'halogènes identiques ou différents,

p est le nombre 0, 1 ou 2.

**2.** 1-arylpyrazoles substitués de formule générale (I) suivant la revendication 1, dans laquelle

$R^1$ représente l'hydrogène, un groupe méthyle, éthyle, n-propyle, isopropyle, n-butyle, isobutyle, sec-butyle ou tertiobutyle,

$R^2$ est un groupe méthyle, éthyle, n-propyle, isopropyle, n-butyle, isobutyle, sec-butyle, tertiobutyle, chlorométhyle, difluorométhyle, difluorochlorométhyle, fluorodichlorométhyle, trifluorométhyle, pentafluoréthyle, pentachloréthyle, fluorotétrachloréthyle, difluorotrichloréthyle, trifluorodichloréthyle, tétrafluorochloréthyle, heptafluoropropyle, chloréthyle, brométhyle, chloropropyle ou bromopropyle,

$R^3$ est l'hydrogène, un groupe acétyle, propionyle ou butyryle,

$R^4$ est l'hydrogène, un groupe méthyle, éthyle, n-propyle, isopropyle, n-butyle, isobutyle, sec-butyle, tertiobutyle, n-pentyle ou isopentyle portant éventuellement 1 à 3 substituants fluoro, chloro, hydroxy, méthoxy, éthoxy, méthylthio ou éthylthio identiques ou différents, un groupe allyle, propényle ou butényle portant chacun 1 à 3 substituants fluoro ou chloro identiques ou différents, un groupe cyclopropyle, cyclopentyle, cyclohexyle, un groupe benzyle ou phényle portant chacun le cas échéant 1 à 5 substituants, identiques ou différents, fluoro, chloro, bromo, cyano, nitro, méthyle, éthyle, méthoxy, éthoxy, méthylthio ou trifluorométhyle, et en outre, au cas où X représente l'oxygène ou le soufre, un équivalent d'un cation sodium, potassium, magnésium, calcium, baryum, cuivre, zinc, manganèse, étain, fer, cobalt ou nickel ou un cation ammonium, phosphonium ou sulfonium portant le cas échéant 1 à 4 substituants identiques ou différents, méthyle, éthyle, n-propyle, isopropyle, n-butyle, isobutyle, sec-butyle, tertiobutyle, benzyle ou phényle,

A est un reste alkylène secondaire à chaîne droite ou ramifiée ayant 1 à 6 atomes de carbone,

X représente l'oxygène, le soufre ou un reste

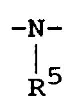

Ar est un groupe phényle portant éventuellement 1 à 5 substituants identiques ou différents ou un groupe 2-pyridyle portant éventuellement 1 à 4 substituants identiques ou différents, en considérant dans chaque cas comme substituants : un radical cyano, nitro, fluoro, chloro, bromo, iodo, méthyle, éthyle, n-propyle, isopropyle, n-butyle, isobutyle, sec-butyle, tertiobutyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorométhyle, pentafluoréthyle, tétrafluoréthyle, trifluorochloréthyle, trifluoréthyle, difluordichloréthyle, trifluorodichloréthyle, pentachloréthyle, trifluorométhoxy, trichlorométhoxy, dichlorofluorométhoxy, difluorochlorométhoxy, chlorométhoxy, dichlorométhoxy, difluorométhoxy, pentafluoréthoxy, tétrafluoréthoxy, trifluorochloréthoxy, trifluoréthoxy, difluorodichloréthoxy, trifluorodichloréthoxy, pentachloréthoxy ou un reste - $S(O)_p$-$R^6$ et

n représente le nombre 0, 1 ou 2,

$R^5$ est l'hydrogène, un groupe méthyle, éthyle, n-propyle, isopropyle, n-butyle, isobutyle, sec-butyle, tertiobutyle, n-pentyle ou isopentyle portant le cas échéant 1 à 3 substituants, identiques ou différents, fluoro, chloro, hydroxy, méthoxy, éthoxy, méthylthio ou éthylthio, un groupe allyle, propényle ou butényle portant le cas échéant 1 à 3 substituants, identiques ou différents, fluoro ou chloro, un groupe propargyle, propynyle, butynyle ou pentynyle, un groupe cyclopropyle, cyclopentyle, cyclohexyle, un groupe benzyle ou phényle portant chacun le cas échéant 1 à 5 substituants, identiques ou différents, fluoro, chloro, bromo, cyano, nitro, méthyle, éthyle, méthoxy, éthoxy, méthylthio ou trifluorométhyle

$R^6$ est un groupe amino, méthylamino, éthylamino, diméthylamino, diéthylamino, fluorodichlorométhyle, difluorométhyle, tétrafluoréthyle, trifluorochloréthyle, trichlorométhyle, trichloréthyle, trifluorométhyle, méthyle ou éthyle,

p est le nombre 0, 1 ou 2.

**3.** 1-arylpyrazoles substitués de formule générale (I) suivant la revendication 1, dans laquelle

$R^1$ est l'hydrogène ou le groupe méthyle

$R^2$ est un groupe trifluorométhyle, dichlorofluorométhyle ou chlorodifluorométhyle,

$R^3$ est l'hydrogène, un groupe acétyle ou propionyle,

$R^4$ est l'hydrogène, un groupe méthyle, éthyle, n-propyle, isopropyle, n-butyle, isobutyle, sec-butyle, tertiobutyle ou benzyle,

A est un reste de formule $-CH_2-$ ; $-CH_2-CH_2-$ ; $-CH_2-CH_2-CH_2-$ ou

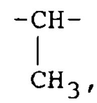

X représente l'oxygène ou un reste

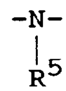

Ar est un groupe phényle portant le cas échéant 1 à 5 substituants, identiques ou différents, fluoro, chloro, bromo, trifluorométhyle ou trifluorométhoxy et

n a la valeur 0, 1 ou 2,

$R^5$ étant un groupe méthyle, éthyle, n-propyle, isopropyle, n-butyle, isobutyle, sec-butyle, tertiobutyle ou un reste $-SO_2-R^6$ et

$R^6$ étant un groupe méthyle, trifluorométhyle, phényle ou p-tolyle.

**4.** Procédé de production de 1-arylpyrazoles de formule générale (I)

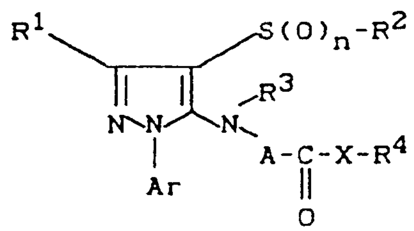

(I)

dans laquelle

$R^1$, $R^2$, $R^3$, $R^4$, A, X, Ar et n ont la définition indiquée dans la revendication 1,

(a) caractérisé en ce que pour obtenir des 1-arylpyrazoles substitués de formule (I),

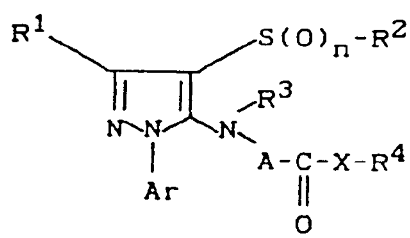

(I)

dans laquelle

$R^1$, $R^2$, $R^3$, $R^4$, A, X, Ar et n ont la définition indiquée ci-dessus,

on fait réagir des 1-arylpyrazoles de formule (II)

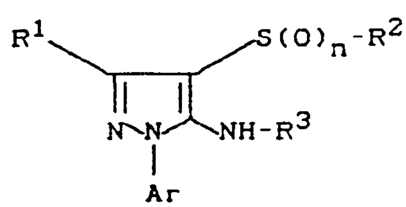

(II)

dans laquelle
$R^1$, $R^2$, $R^3$, Ar et n ont la définition indiquée ci-dessus,
avec des agents alkylants de formule (III),

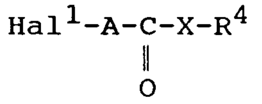

(III)

dans laquelle
$Hal^1$ représente le chlore, le brome ou l'iode et $R^4$, A et X ont la définition indiquée ci-dessus, le cas échéant en présence d'un diluant et en la présence éventuelle d'une substance auxiliaire de réaction ; ou bien
(b) pour obtenir des 1-arylpyrazoles de formule (Ib),

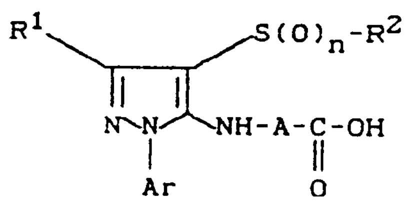

(Ib)

dans laquelle
$R^1$, $R^2$, A, Ar et n ont la définition indiquée ci-dessus,
on saponifie des 1-arylpyrazoles de formule (Ia),

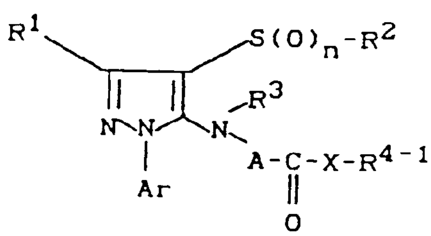

(Ia)

dans laquelle
$R^{4-1}$ représente un groupe alkyle ou un groupe hydroxyalkyle à chaîne droite ou ramifiée ayant chacun 1 à 12 atomes de carbone, un groupe alkoxyalkyle ou un groupe alkylthioalkyle à chaîne droite ou ramifiée ayant chacun 2 à 12 atomes de carbone, un groupe alcényle ou un groupe alcynyle à chaîne droite ou ramifiée ayant chacun 2 à 12 atomes de carbone, un groupe halogénalkyle à chaîne droite ou ramifiée ayant 1 à 8 atomes de carbone et 1 à 17 atomes d'halogènes identiques ou différents, un groupe halogénalkyle à chaîne droite ou ramifiée ayant 2 à 8 atomes de carbone et 1 à 15 atomes d'halogènes identiques ou différents, un groupe cycloalkyle de 3 à 7 atomes de carbone portant le cas échéant un ou plusieurs substituants alkyle en $C_1$ à $C_4$ à chaîne droite ou ramifiée, identiques ou différents, ou un groupe phényle, benzyle ou phényléthyle portant chacun le cas échéant dans la partie phényle un ou plusieurs substituants identiques ou différents, en considérant dans chaque cas comme substituants de la partie phényle : un halogène, un groupe cyano, nitro, un groupe alkyle, alkoxy ou alkylthio à chaîne droite ou ramifiée ayant chacun 1 à 4 atomes de carbone, un groupe halogénalkyle, halogénalkoxy ou halogénalkylthio à chaîne droite ou ramifiée ayant chacun 1 à 4 atomes de carbone et 1 à 9 atomes d'halogènes identiques ou différents, un groupe alkoxycarbonyle ou un groupe alkoximinoalkyle à chaîne droite ou ramifiée ayant chacun 1 à 4 atomes de carbone dans les parties alkyle individuelles ainsi qu'un groupe phényle portant le cas échéant un ou plusieurs substituants halogéno et/ou alkyle en $C_1$ à $C_4$ à chaîne droite ou ramifiée identiques ou différents ;

$R^1$, $R^2$, $R^3$, A, X, Ar et n — ont la définition indiquée ci-dessus,

avec des acides ou des bases comme catalyseur en présence d'eau ainsi que, le cas échéant, en présence d'un diluant ;

(c) pour l'obtention de 1-arylpyrazoles substitués de formule (Ic) ,

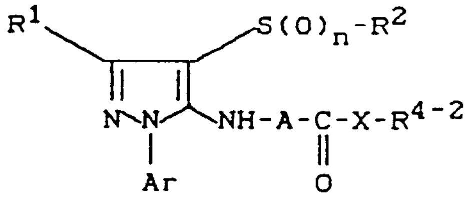

(Ic)

dans laquelle

$R^{4-2}$ — représente l'hydrogène, un groupe alkyle ou un groupe hydroxyalkyle à chaîne droite ou ramifiée ayant chacun 1 à 12 atomes de carbone, un groupe alkoxyalkyle ou un groupe alkylthioalkyle à chaîne droite ou ramifiée ayant chacun 2 à 12 atomes de carbone, un groupe alcényle ou un groupe alcynyle à chaîne droite ou ramifiée ayant chacun 2 à 12 atomes de carbone, un groupe halogénalkyle à chaîne droite ou ramifiée ayant 1 à 8 atomes de carbone et 1 à 17 atomes d'halogènes identiques ou différents, un groupe halogénalcényle à chaîne droite ou ramifiée ayant 2 à 8 atomes de carbone et 1 à 15 atomes d'halogènes identiques ou différents, un groupe cycloalkyle de 3 à 7 atomes de carbone portant le cas échéant un ou plusieurs substituants alkyle en $C_1$ à $C_4$ à chaîne droite ou ramifiée, identiques ou différents, ou un groupe phényle, benzyle ou phényléthyle portant chacun le cas échéant dans la partie phényle un ou plusieurs substituants identiques ou différents, en considérant dans chaque cas comme substituants de la partie phényle : un radical halogéno, cyano, nitro, un radical alkyle, alkoxy ou alkylthio à chaîne droite ou ramifiée ayant chacun 1 à 4 atomes de carbone, un radical halogénalkyle, halogénalkoxy ou halogénalkylthio à chaîne droite ou ramifiée ayant chacun 1 à 4 atomes de carbone et 1 à 9 atomes d'halogènes identiques ou différents, un radical alkoxycarbonyle ou alkoximinoalkyle à chaîne droite ou ramifiée ayant chacun 1 à 4 atomes de carbone dans les parties alkyle individuelles ainsi qu'un groupe phényle portant éventuellement un ou plusieurs substituants, identiques ou différents, halogéno et/ou alkyle à chaîne droite ou ramifiée ayant 1 à 4 atomes de carbone ;
et

$R^1$, $R^2$, A, X Ar et n — ont la définition indiquée ci-dessus, on fait réagir des 1-arylpyrazoles substitués de formule (Ib),

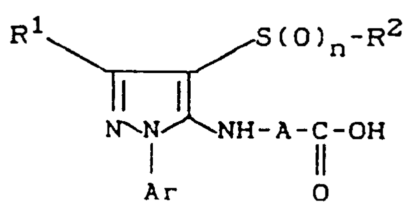

(Ib)

dans laquelle
$R^1$, $R^2$, A et n ont la définition indiquée ci-dessus,
avec des alkols, des thiols ou des composés aminés de formule (IV),

$R^{4-2}$-X-H (IV)

dans laquelle

$R^{4-2}$ et X ont la définition indiquée ci-dessus, en présence d'un catalyseur d'acylation ainsi que, le cas échéant, en présence d'un diluant et en la présence éventuelle d'une substance auxiliaire de réaction ; ou bien

(d) en vue d'obtenir des 1-arylpyrazoles substitués de formule (Id),

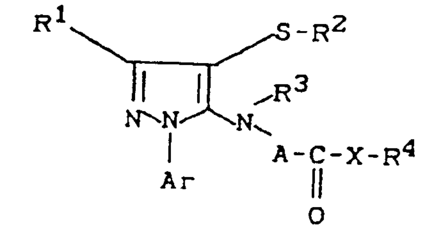

(Id)

dans laquelle

$R^1$, $R^2$, $R^3$, $R^4$, A, X et Ar ont la définition indiquée ci-dessus,

on fait réagir des 1-arylpyrazoles non substitués en position 4 de formule (V),

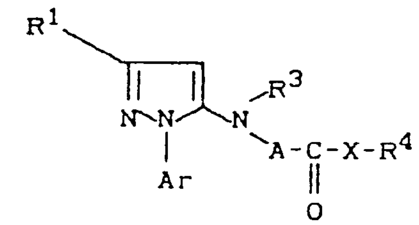

(V)

dans laquelle

$R^1$, $R^3$, $R^4$, A, X et Ar ont la définition indiquée ci-dessus,

avec des halogénures de sulfényle de formule (VI),

$R^2$-S-$Hal^2$ (VI)

dans laquelle

$Hal^2$ représente le chlore ou le brome et

$R^2$ a la définition indiquée ci-dessus,

le cas échéant en présence d'un diluant et en la présence éventuelle d'une substance auxiliaire de réaction ; ou bien

(e) en vue d'obtenir des 1-arylpyrazoles substitués de formule (Ie),

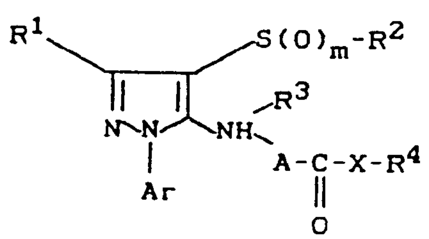

(Ie)

dans laquelle

$R^1$, $R^2$, $R^3$, $R^4$, A, X et Ar ont la définition indiquée ci-dessus, et

m représente le nombre 1 ou 2,

on fait réagir des 1-arylpyrazoles substitués de formule (Id),

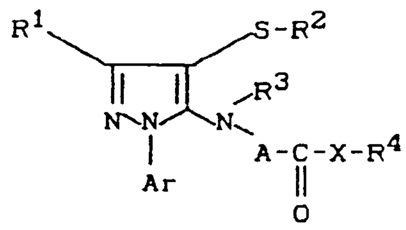

(Id)

dans laquelle
$R^1$, $R^2$, $R^3$, $R^4$, A, X et Ar ont la définition indiquée ci-dessus,
avec des agents oxydants, le cas échéant en présence d'un diluant et en la présence éventuelle d'un catalyseur ; ou bien
(f) en vue d'obtenir des 1-arylpyrazoles substitués de formule (If),

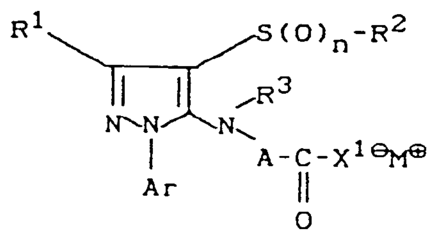

(If)

dans laquelle
$X^1$ représente l'oxygène ou le soufre,
$M^{\oplus}$ est un cation lié à la façon d'un sel et
$R^1$, $R^2$, $R^3$, A, Ar et n ont la définition indiquée ci-dessus,
on fait réagir des 1-arylpyrazoles substitués de formule (Ig),

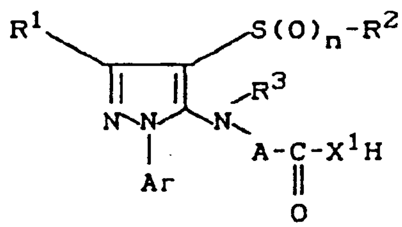

(Ig)

(Ig)

dans laquelle
$R^1$, $R^2$, $R^3$, A, $X^1$, Ar et n ont la définition indiquée ci-dessus,
avec des bases inorganiques ou organiques, le cas échéant en présence d'un diluant.

**5.** Compositions pesticides, caractérisées par une teneur en au moins un 1-arylpyrazole de formule (I) suivant la revendication 1.

**6.** Compositions insecticides, caractérisées par une teneur en au moins un 1-arylpyrazole de formule (I) suivant la revendication 1.

**7.** Procédé pour combattre des insectes, caractérisé en ce qu'on fait agir des 1-arylpyrazoles de formule (I) suivant la revendication 1 sur les insectes et/ou sur leur milieu, excepté la lutte contre des ectoparasites.

**8.** Utilisation de 1-arylpyrazoles de formule (I) suivant la revendication 1 pour combattre des insectes, à l'exception de la lutte contre des ectoparasites.

**9.** Procédé de préparation de compositions insecticides, caractérisé en ce qu'on mélange des 1-arylpyrazoles de formule (I) suivant la revendication 1 avec des diluants et/ou des agents tensio-actifs.

**10.** Utilisation de 1-arylpyrazoles de formule (I) suivant la revendication 1 pour la préparation de compositions destinées à la lutte contre des ectoparasites.